# EUROPEAN PATENT SPECIFICATION

(11) **EP 1 609 867 B1**
(45) Date of publication and mention of the grant of the patent: **29.09.2010**
(21) Application number: 05000339.1
(22) Date of filing: 10.01.2005
(51) Int. Cl.: C12N 15/63, C12N 15/67, C12N 15/71, C12N 15/72, C12N 15/73, C12N 5/10

(54) **Nucleic acid construct, expression vector and method for enhancing the production of recombinant protein**
Nukleinsäurekonstruct, Expressionsvektor und Verfahren zur Steigerung der Hergestellten Menge von rekombinantem Eiweiss
Construction d'acide nucléique, vecteur et procédé pour élever la production de protéine de recombinaison

(30) Priority: 25.06.2004 CN 200410061855
(43) Date of publication of application: 28.12.2005
(73) Proprietor: Feng Chia University, Taichung City, Taiwan (TW); Taichung District Agriculture Research and Extension Station, Council of Agriculture, Chang-Hwa County (TW)
(72) Inventor: Chao, Yun-Peng, Taichung City (TW); Wang, Zei-Wen, Tainan City (TW); Chen, Po-Ting, Tainan City (TW); Chen, Yu-Hsin, Taichung City (TW)
(74) Representative: Fleuchaus, Andrea

(56) References cited:
- SUN A L ET AL: "Fusion expression of human pro-urokinase with E. coli thioredoxin." BIOCHEMISTRY AND MOLECULAR BIOLOGY INTERNATIONAL. OCT 1998, vol. 46, no. 3, October 1998 (1998-10), pages 479-486, XP009050336 ISSN: 1039-9712
- YASUKAWA T ET AL: "Increase of solubility of foreign proteins in Escherichia coli by coproduction of the bacterial thioredoxin." THE JOURNAL OF BIOLOGICAL CHEMISTRY. 27 OCT 1995, vol. 270, no. 43, 27 October 1995 (1995-10-27), pages 25328-25331, XP002335492 ISSN: 0021-9258 & EP 0 768 382 A (HSP RESEARCH INSTITUTE, INC; THE INSTITUTE OF PHYSICAL & CHEMICAL RESE) 16 April 1997 (1997-04-16)
- FREY A D ET AL: "Expression of Alcaligenes eutrophus flavohemoprotein and engineered Vitreoscilla hemoglobin-reductase fusion protein for improved hypoxic growth of Escherichia coli." APPLIED AND ENVIRONMENTAL MICROBIOLOGY. JAN 2000, vol. 66, no. 1, January 2000 (2000-01), pages 98-104, XP002335493 ISSN: 0099-2240
- FREY ALEXANDER D ET AL: "Bacterial hemoglobins and flavohemoglobins: versatile proteins and their impact on microbiology and biotechnology." FEMS MICROBIOLOGY REVIEWS. OCT 2003, vol. 27, no. 4, October 2003 (2003-10), pages 525-545, XP002335494 ISSN: 0168-6445

### SEQUENCE LISTING

<110> FENG CHIA UNIVERSITY TAICHUNG DISTRICT AGRICULTURE RESEARCH AND EXTENSION STATION, COUNCIL OF AGRICULTURE
<120> NUCLEIC ACID CONSTRUCT AND EXPRESSION VECTOR FOR ENHANCING THE PRODUCTION OF RECOMBINANT PROTEIN, AND METHOD FOR THE MASSIVE PRODUCTION OF RECOMBINANT PROTEIN
<130> PE-26863-EU
<160> 8
<170> Patent In version 3.2
<210> 1
   <211> 27
   <212> DNA
   <213> Artificial
<220>
   <223> artificial forward primer of Vistreoscilla hemoglobin gene
<400> 1
   aagggatcca tgttagacca gcaaacc 27
<210> 2
   <211> 27
   <212> DNA
   <213> Artificial
<220>
   <223> artificial reverse primer of Vitreoscilla hemoglobin gene
<400> 2
   atagtcgacc caagttttgg caacagc 27
<210> 3
   <211> 29
   <212> DNA
   <213> Artificial
<220>
   <223> artificial forward primer of E. coli thioredoxin gene
<400> 3
   ccgaattcaa ccacacctat ggtgtatgc 29
<210> 4
   <211> 25
   <212> DNA
   <213> Artificial
<220>
   <223> artificial reverse primer of E. coli thioredoxin gene
<400> 4
   ggggatccgc tgcaaggcga ttaag 25
<210> 5
   <211> 28
   <212> DNA
   <213> Artificial
<220>
   <223> artificial primer of human interferon alpha2 gene
<400> 5
   tgctctcata tgttgatctg cctcaaac 28
<210> 6
   <211> 33
   <212> DNA
   <213> Artificial
<220>
   <223> artificial reverse primer of human interferon alpha2 gene
<400> 6
   cgtgctcgag ttattattcc ttacttctta aac 33
<210> 7
   <211> 28
   <212> DNA
   <213> Artificial
<220>
   <223> artificial forward primer of aspartase gene
<400> 7
   cacaggatcc acaacattcg tatcgaag 28
<210> 8
   <211> 29
   <212> DNA
   <213> Artificial
<220>
   <223> artificial reverse primer of aspartase gene
<400> 8
   acgagtcgac ttcgctttca tcagtatag 29

## Description

This invention relates to nucleic acid constructs and expression vectors for enhancing the production of recombinant polypeptides/proteins, and methods for the massive production of recombinant polypeptides/proteins, in which a first nucleic acid sequence encoding thioredoxin and a second nucleic acid sequence encoding hemoglobin are cloned into a host cell, thereby enhancing the capability of the thus formed recombinant host cell in producing a selected gene product and assisting the recombinant host cell in relieving intracellular stress due to overproduction of the gene product.

The "production of recombinant polypeptides/ proteins" is a very important genetic engineering technique in the field of biotechnology. The basic principle involves the cloning of a target gene capable of expressing a desired gene product (*e*.*g*., industrial and agricultural enzymes, therapeutic proteins, interferons, interleukins, hormones, growth hormones, antigenic polypeptides, antibodies) into a suitable vector, and the subsequent transfer of the resultant recombinant vector into a competent host cell. The thus formed recombinant host cell can be cultured in a suitable culture medium and under suitable culture conditions, and expression of the target gene can be induced at an opportune time so as to achieve the object of massive production of the desired gene product.

To date, in the production of recombinant polypeptides/proteins, *Escherichia coli* cells are the most widely used and the most effective host cells, and many types of plasmid vectors can be developed from this bacterial species, including high-copy-number plasmids (such as ColE1), medium-copy-number plasmids (such as p15A), low-copy-number plasmids (such as pSC101), and plasmids (such as R1) that control the copy number thereof by temperature (S.C. Makrides et al. (1996), Microbiol. Rev., 60:512-538). The plasmid vectors thus developed and constructed generally have an inducible artificial promoter. If a target gene is cloned downstream of the artificial promoter, the object of controlling the expression of the target gene can be achieved.

In general, the most commonly used artificial promoters include *lac, trp, tac, trc, araBAD,* λ*P_{R}P_{L},* and T7 promoters, and these promoters may be induced by the addition of isopropyl-β-D-thiogalactopyranoside (IPTG), lactose, arabinose, a change in temperature or the like (S.C. Makrides et al. (1996), Microbiol. Rev., 60: 512-538). On the other hand, a cloned target gene can be directly cloned into a vector if it contains a constitutive promoter. When such a target gene is used in the construction of a recombinant vector, normally it is not necessary to elicit the production of recombinant polypeptides/proteins by induction methods.

Regardless of which method is used to clone a target gene into a plasmid vector, to produce recombinant polypeptides/proteins, a recombinant plasmid vector containing a cloned target gene must be transferred into a host cell, and the transformed host cell thus formed serves as a factory for the production of recombinant polypeptides/proteins. However, there are many factors that may cause instability and thus loss of the plasmid vector harbored within the transformed host cell. These factors include, for instance, constituents of culture media, conditions for cultivating host cells, characteristics of plasmids and host cells themselves, toxicity of the expressed polypeptide/protein products, *etc.* Therefore, in view of the stability problems of plasmid vectors, one may consider employing homologous recombination, as well as attachment mediated by bacteriophages and transposition mediated by transposons, to insert target gene(s) into cell chromosome(s)(A. Haldimann et al. (2001), J Bacteriol., 183: 6384-6393).

According to existing knowledge and technology in today's biotechnology field, theoretically, almost all the genes derived from different biological sources can be expressed in *E. coli* cells, and relevant manipulating procedures have been well developed. However, when one intends to put such manipulating procedures into industrial application, there arise many problems that need to be solved. For example, transformed bacterial cells will oftentimes bear a considerable metabolic burden when they are induced to overproduce recombinant polypeptides/proteins. The so-called "metabolic burden" refers to a condition in which the growth of transformed host cells is retarded due to massive production of recombinant polypeptides/proteins within said cells, thereby triggering stress responses in said cells (T. Schweder et al. (2002), Appl. Microbiol. Biotechnol., 58:330-337). As a result, a large amount of heat shock proteins are produced within the cells, leading to the disintegration and proteolytic attack of the produced recombinant polypeptides/proteins (H. Bahl et al. (1987), Gene Dev., 1:57-64). The production of heat shock proteins may also result in retarded growth of the cells (C.G. Kurland et al. (1996), Mol. Microbiol., 21:1-4), or may cause damage to the cellular rRNAs, thereby resulting in ribosome disruption and death of the cells (H. Dong et al. (1995), J. Bacteriol., 177:1497-1504).

It is worth noting that recombinant polypeptides/proteins overproduced by transformed host cells are likely to induce stress responses in the transformed host cells, regardless of whether or not they are relevant to the metabolic growth of the host cells or whether they have toxicity. Therefore, the object of massive production of recombinant polypeptides/proteins can hardly be achieved.

Since the production of recombinant polypeptides/proteins is critical to the competitiveness in the biotechnology industry, how to overcome the aforesaid problems and to achieve the object of high production of recombinant polypeptides/proteins is an extremely important subject of research and development in the biotechnology industry.

On the other hand, maintaining an adequate supply of oxygen to aerobically growing cell cultures is a central problem in a variety of bioprocesses.

*Vitreoscilla* bacteria are a group of filamentous aerobic bacteria that can grow in oxygen-poor environments. Growth of bacteria of this genus under hypoxic conditions results in a several-fold induction of synthesis of a homodimeric soluble heme protein (subunit MW 15,775). Said heme protein was later proven to be a bacterial hemoglobin, which has a remarkable spectral (Webster et al. (1974), Journal of Biological Chemistry 249:4257-4260), structural (Wakabayashi et al. (1986), Nature, 322:481-483), and kinetic (Orii et al. (1986), Journal of Biological Chemistry 261:2978-2986) homology with eucaryotic hemoglobins (cf: US 5,049,493).

In the previous studies by C. Khosla *et al*., the hemoglobin gene of *Vitreoscilla sp.* was cloned into *E. coli* cells and expressed (C. Khosla and J.E. Bailey (1988), Mol. Gen. Genet., 214:158-161), and the growth properties of recombinant *E. coli* cells carrying the *Vitreoscilla* hemoglobin gene in the fermentation culture process under hypoxic conditions were noticeably enhanced as compared to wild-type *E. coli* cells, e.g., faster growth, increased total cell mass (C. Khosla and J.E. Bailey (1988), Nature, 331:633-635).

US 5,049,493 issued to C. Khosla et al. discloses nucleotide sequences of the *Vitreoscilla* hemoglobin which include a structural gene encoding the protein and a gene promoter/regulator which is useful in subjecting the transcription/translation of DNA sequences to selective regulation by external control, and plasmid vectors containing those nucleotide sequences, which are useful in enhancing growth characteristics of cells and increasing production of various proteins and metabolites of cells. The *Vitreoscilla* hemoglobin can enhance the growth and product synthesis characteristics of aerobic organisms in environments with sufficient and reduced or low levels of oxygen.

It is further reported in literature that, during the fermentation culture process under hypoxic conditions, recombinant *E. coli* cells that produce *Vitreoscilla* hemoglobin can effectively increase the amount of proteins generated by the cells per se (C. Khosla et al. (1990), BiolTechnology, 8:849-853), and the production of recombinant α-amylase (M. Khosravi et al. (1990), Plasmid, 24:190-194). In addition, when *Bacillus subtilis* cells and Chinese hamster ovary cells are used as host cells, under hypoxic conditions, recombinant cells containing *Vitreoscilla* hemoglobin therein can likewise produce a relatively high amount of recombinant proteins (P.T. Kallio and J.E.Bailey (1996), Biotechnol. Prog., 12:31-39; G.J. Pendse and J.E. Bailey (1994), Biotehnol. Bioeng., 44:1367-1370).

Interestingly, as compared to wild-type strains, the production rate of NAD(P)H in recombinant *E. coli* cells that produce *Vitreoscilla* hemoglobins is reduced by 2.4-fold. This result indicated that recombinant cells which produce *Vitreoscilla* hemoglobins are in an oxidized state (P.S. Tsai et al. (1995), Biotechnol Bioeng., 49: 347-354).

In generally, under normal cell physiological conditions, *E. coli* cells are in a reduced state. Therefore, proteins located within the cytoplasm do not easily form a disulfide bond. However, it is currently known that there are two intracellular enzymes, such as ribonucleotide reductase and oxidative response transcription factor (OxyR), which can form a disulfide bond during the reaction cycle they participate in. Such a protein disulfide bond is transitionally formed during the reaction path mediated by thioredoxin and glutathione/glutaredoxin in the cells (A. Aberg et at (1989), J Biol Chem., 264:12249-12252; M. Zheng et at (1998), Science, 279:1718- 1721). It has been reported that overproduction of thioredoxin in E. coil can promote the reduced state in the cells, which is conducive to the solubility of eukaryotic proteins produced (E.R. LaVallie et at (1993), Bio/Technology, 11:187-193; T. Yasukawa et al. (1995), J Biol Chem., 270:25328-25331).

T. Yasukawa et al. (1995) discloses that the coproduction of Trx increases solubility of several parallel expressed vertebrate proteins.

US 2003/0167524 A1 discloses methods for the production of recombinant proteins in association with oil bodies. The recombinant proteins may be first and/or second recombinant polypeptides, multimericprotein-complexes, heteromultimeric-protein-complexes, multimeric-fusion-proteins, heteromultimeric-fusion-proteins, immunoglobulinpolypeptide-chains, immunoglobins, redox-fusion-polypeptides, and/or thioredoxin-related proteins, of which the first recombinant polypeptide is a thioredoxin, whereas the second recombinant polypeptide is a thioredoxin-reductase.

Frey & Kallio, ((2003) FEMS Microbiology Reviews 27, 525-545) being a review article teaches the present general knowledge regarding bacterial and flavohemoglobins and discusses potential uses of these hemoglobins, and also of VHb (Vitreoscilla hemoglobin) in biotechnology. However, to the applicants' knowledge, to achieve the object of massive production of recombinant proteins using technology existing in the current biotechnological field is still difficult. Therefore, there is a need in the art for the development of new technology to improve the production of recombinant polypeptides/proteins.

When overproduction of a desired recombinant protein is carried out in a transformed host cell, it is possible to induce the so-called stress response or metabolic burden is induced in the transformed host cell, thereby rendering the transformed host cell unable to overproduce the recombinant protein. Therefore, the applicants attempted to provide an effective and general solution so as to achieve the object of massive production of recombinant proteins.

According to a first aspect, this invention provides a method for enhancing the production of a selected gene product in a recombinant host cell, which comprises:
(a) cloning into a host cell a gene sequence encoding the selected gene product, a first nucleic acid sequence encoding thioredoxin, and a second nucleic acid sequence encoding Vitreoscilla hemoglobin, so as to form a recombinant host cell;
(b) cultivating a recombinant host cell formed from step (a) in a suitable medium, so as to allow the expression of said gene sequence; and
(c) harvesting the expressed gene product.

According to a second aspect, this invention provides a recombinant host cell capable of expressing a selected gene product, comprising therein a gene sequence encoding the selected gene product, a first nucleic acid sequence encoding thioredoxin, and a second nucleic acid sequence encoding Vitreoscilla hemoglobin.

According to a third aspect, this invention provides a nucleic acid construct for the implementation of the aforesaid method and the formation of the aforesaid recombinant host cell, comprising a first nucleic acid sequence encoding thioredoxin, and a second nucleic acid sequence encoding Vitreoscilla hemoglobin. This invention also provides a vector carrying the nucleic acid construct.

The techniques according to this invention can be used to improve the properties of transformed host cells that produce recombinant proteins, such as the abilities to increase the protein production, to resist protein toxicity, to protect the proteins produced within the cells from disintegration, and to maintain the integrity of the ribosomes in the cells.

According to this invention, the efficiency of producing recombinant proteins by transformed host cells under aerobic conditions can be enhanced, fermentation culture time can be effectively shortened, and transformed host cells can be cultivated at a higher cell density.

In addition, the practice of this invention is not limited to the use of specific host cells. In fact, this invention can be applied to a diversity of different host cells, including bacteria, yeasts, fungi, plant cells, insect cells, mammalian cells, etc., and can be used to form different kinds of proteins, including proteins present in the cytoplasm or the periplasm, proteins present on cell membranes or extracellular proteins, and enzymes available for use in industry and in agriculture, food industry, environmental industry, aquaculture and animal husbandry, particularly pharmaceutical proteins and peptides, such as interferons, human and animal hormones, immunogenic antigens, and antibodies.

Other features and advantages of the present invention will become apparent in the following detailed description of the preferred embodiment with reference to the accompanying drawings, of which:

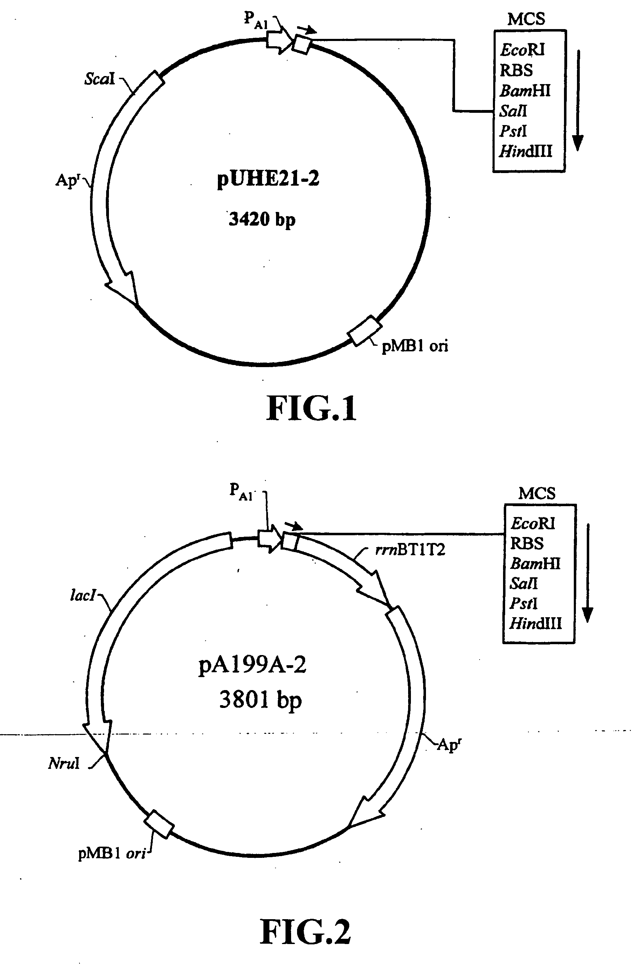

Figure 1 shows the construction of plasmid pUHE21-2, which includes: pMB1 ori, the replication origin of plasmid pMB1 ; P_{A1}, T7 A1 promoter; Ap^{r}, ampicillin resistance gene; *Sca*I, the cutting site of restriction enzyme *Sca*I; and multiple cloning sites (MCS), in which the ribosome binding site (RBS) and the restriction enzyme cutting sites contained therein are labeled within an MCS frame box ;
Figure 2 shows the construction of plasmid pA199A-2, which includes: pMB1 ori, the replication origin of plasmid pMB1 ; P_{A1}, T7 A1 promoter; *lacl, lac* repressor protein gene; *rrnB*T1T2, *rrnB* gene transcriptional termination site; Ap^{r}, ampicillin resistance gene; *Nrul,* the cutting site of restriction enzyme *Nru*I; and multiple cloning sites (MCS), in which the ribosome binding site (RBS) and the restriction enzyme cutting sites contained therein are labeled within an MCS frame box;

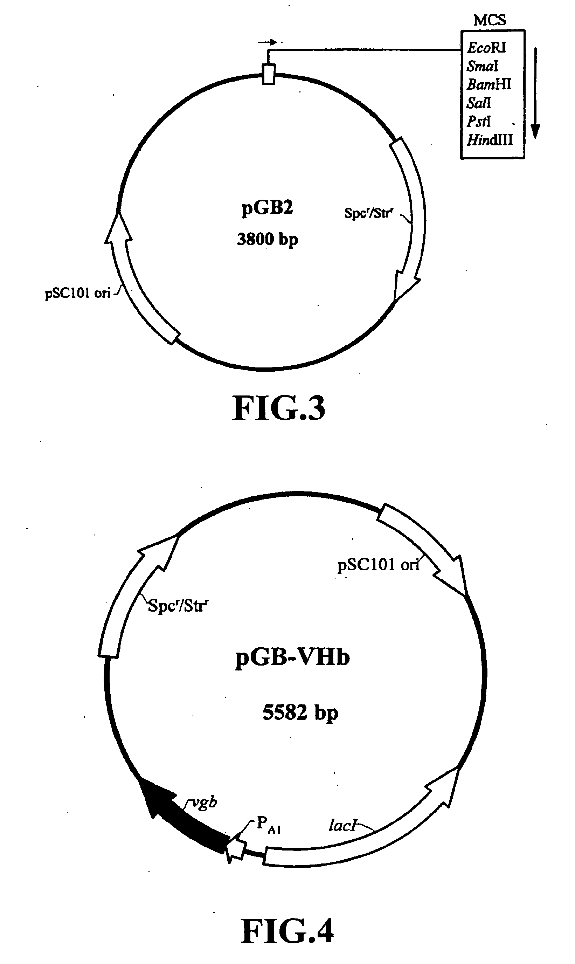

Figure 3 shows the construction of plasmid pGB2, which contains: pSC101 ori, the replication origin of plasmid pSC101; Spc^{r}/Str^{r}, spectinomycin/streptomycin resistance gene; and multiple cloning sites (MCS), in which the restriction enzyme cutting sites contained therein are labeled within an MCS frame box;
Figure 4 shows the construction of plasmid pGB-VHb, which contains: pSC101 ori, the replication origin of plasmid pSC101; *vgb, Vitreoscilla* hemoglobin structural gene; P_{A1}, T7 A1promoter; *lacl, lac* repressor protein gene; and Spc^{r}/Str^{r}, spectinomycin/streptomycin resistance gene;

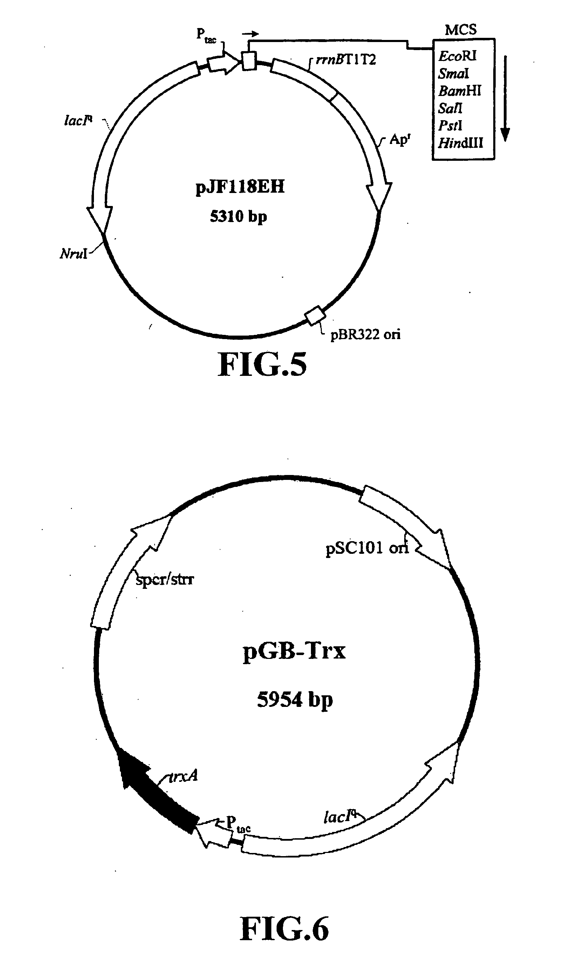

Figure 5 shows the construction of plasmid pJF118EH, which contains: pBR322 ori, the replication origin of plasmid pBR322; P_{tac}, *tac* promoter; *lacl*^{q}, *lac* repressor protein gene; *rrnB*T1T2, *rrnB* gene transcriptional termination site; *Nru*I*,* the cutting site of restriction enzyme *Nru*I; Ap^{r}, ampicillin resistance gene; and multiple cloning sites (MCS), in which the restriction enzyme cutting sites contained therein are labeled within an MCS frame box;
Figure 6 shows the construction of plasmid pGB-Trx, which contains: pSC101 ori, the replication origin of plasmid pSC101; *trxA*, thioredoxin structural gene; P_{tac}, *tac* promoter; *lacl*^{q}, *lac* repressor protein gene; and Spc^{r}/Str^{r}, spectinomycin/streptomycin resistance gene;

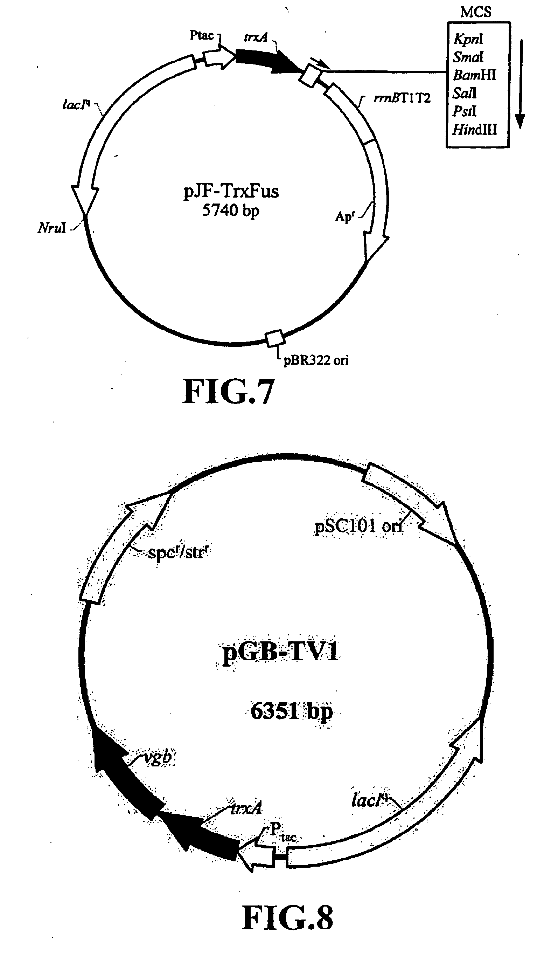

Figure 7 shows the construction of plasmid pJF-TrxFus, which contains: pBR322 ori, the replication origin of plasmid pBR322; P_{tac}, *tac* promoter; *trxA*, thioredoxin structural gene (excluding the stop codon); *lacI*^{q}, *lac* repressor protein gene; *Nru*I, the cutting site of restriction enzyme *Nru*I; *rrnB*T1T2, *rrnB* gene transcriptional termination site; Ap^{r}, ampicillin resistance gene; and multiple cloning sites (MCS), in which the restriction enzyme cutting sites contained therein are labeled within an MCS frame box;
Figure 8 shows the construction of plasmid pGB-TV1, which contains: pSC101 ori, the replication origin of plasmid pSC101; *trxA*, thioredoxin structural gene; *vgb, Vitreoscilla* hemoglobin structural gene; P_{tac}, *tac* promoter; *lacI*^{q}, *lac* repressor protein gene; and Spc^{r}/Str^{r}, spectinomycin/streptomycin resistance gene;

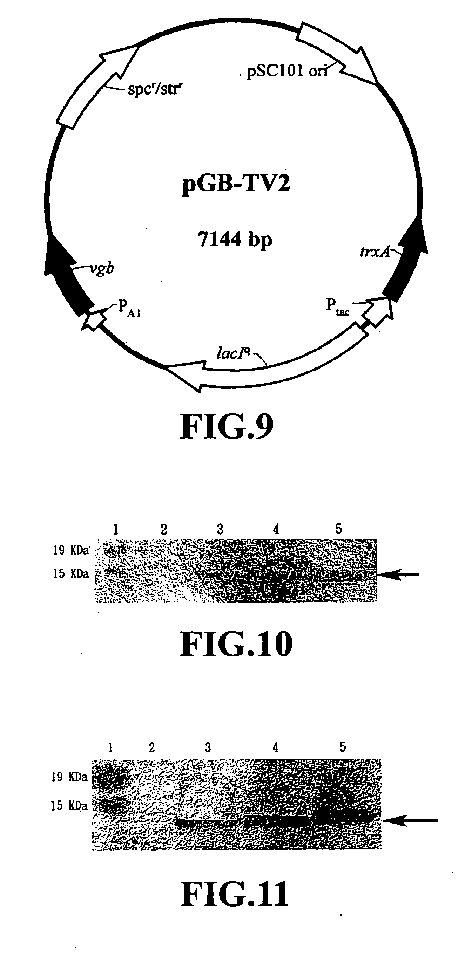

Figure 9 shows the construction of plasmid pGB-TV2, which contains: pSC101 ori, the replication origin of plasmid pSC101; *trxA*, thioredoxin structural gene; *vgb, Vitreoscilla* hemoglobin structural gene; P_{tac}, *tac* promoter; P_{A1}, T7 A1 promoter; *lacl*^{q}, *lac* repressor protein gene; and Spc^{r}/Str^{r}, spectinomycin/streptomycin resistance gene;
Figure 10 is a Western blot showing the immunodetection of *Vitreoscilla* hemoglobin produced by an IPTG-induced recombinant *E. coli* strain BL21(DE3)/pGB-VHb using a primary antibody against *Vitreoscilla* hemoglobin, wherein lane 1: protein standards (ProSieve® Color Protein Marker, Cambrex BioScience); lane 2: a protein sample of the strain without IPTG induction; lane 3: a protein sample of the strain induced with 30 µM IPTG; lane 4: a protein sample of the strain induced with 100 µM IPTG; lane 5: a protein sample of the strain induced with 500 µM IPTG; and the arrow indicates the position of *Vitreoscilla* hemoglobin;
Figure 11 is a Western blot showing the immunodetection of thioredoxin produced by an IPTG-induced recombinant *E. coli* strain BL21(DE3)/pGB-Trx using a primary antibody against thioredoxin, wherein lane 1: protein standards (ProSieve® Color Protein Marker, Cambrex BioScience); lane 2: a protein sample of the strain without IPTG induction; lane 3: a protein sample of the strain induced with 30 µM IPTG; lane 4: a protein sample of the strain induced with 100 µM IPTG; lane 5: a protein sample of the strain induced with 500 µM IPTG; and the arrow indicates the position of thioredoxin;

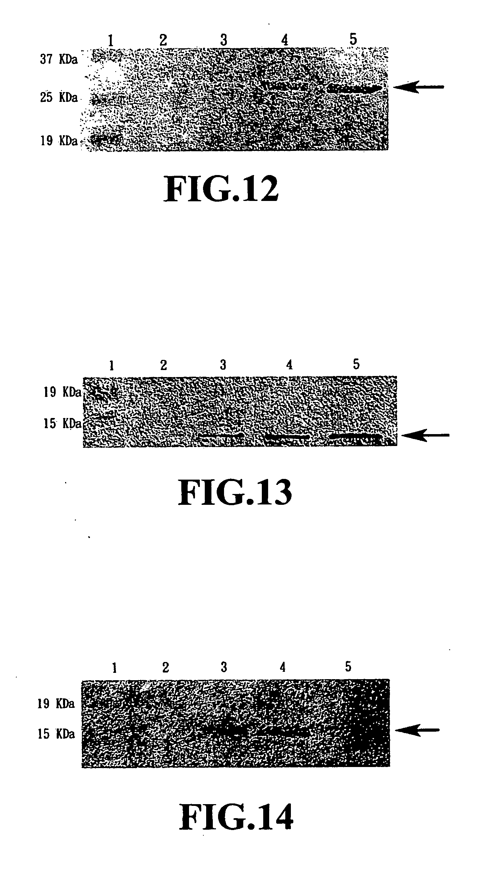

Figure 12 is a Western blot showing the immunodetection of a fusion protein of thioredoxin and *Vitreoscilla* hemoglobin produced by an IPTG-induced recombinant *E. coli* strain BL21(DE3)/pGB-TV1 using a primary antibody against thioredoxin, wherein lane 1: protein standards (ProSieve® Color Protein Marker, Cambrex BioScience); lane 2: a protein sample of the strain without IPTG induction; lane 3: a protein sample of the strain induced with 30 µM IPTG; lane 4: a protein sample of the strain induced with 100 µM IPTG; lane 5: a protein sample of the strain induced with 500 µM IPTG; and the arrow indicates the position of the fusion protein of thioredoxin and *Vitreoscilla* hemoglobin;
Figure 13 is a Western blot showing the immunodetection of thioredoxin produced by an IPTG-induced recombinant *E. coli* strain BL21(DE3)/pGB-TV2 using a primary antibody against thioredoxin, wherein lane 1: protein standards (ProSieve® Color Protein Marker, Cambrex BioScience); lane 2: a protein sample of the strain without IPTG induction; lane 3: a protein sample of the strain induced with 30 µM IPTG; lane 4: a protein sample of the strain induced with 100 µM IPTG; lane 5: a protein sample of the strain induced with 500 µM IPTG; and the arrow indicates the position of thioredoxin;
Figure 14 is a Western blot showing the immunodetection of *Vitreoscilla* hemoglobin produced by an IPTG-induced recombinant *E. coli* strain BL21(DE3)/pGB-TV2 using a primary antibody against *Vitreoscilla* hemoglobin, wherein lane 1: protein standards (ProSieve® Color Protein Marker, Cambrex BioScience); lane 2: a protein sample of the strain without IPTG induction; lane 3: a protein sample of the strain induced with 30 µM IPTG; lane 4: a protein sample of the strain induced with 100 µM IPTG; lane 5: a protein sample of the strain induced with 500 µM IPTG; and the arrow indicates the position of *Vitreoscilla* hemoglobin;

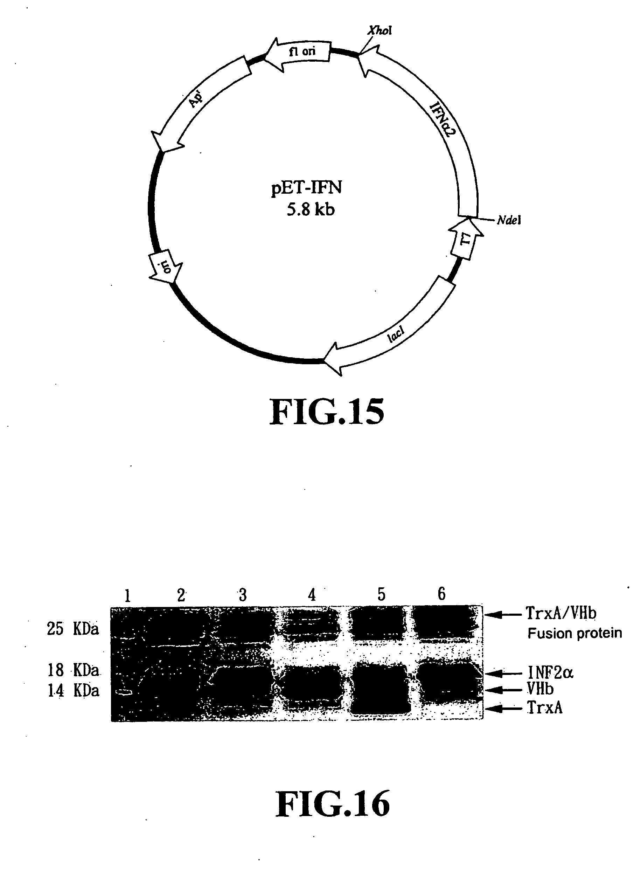

Figure 15 shows the construction of plasmid pET-IFN, which contains: ori, pBR322 replication origin; f1 ori, f1 replication origin; lFNα2, human interferon α2 gene; T7, T7 promoter; *Nde*I, the cutting site of restriction enzyme *Nde*I; *Xho*I, the cutting site of restriction enzyme *Xho*I; *lacl, lac* repressor protein gene; and Ap^{r}, ampicillin resistance gene;
Figure 16 shows a protein electrophoresis graph, wherein lane 1: protein standards (MBI Fermentas); lane 2: a protein sample of a control strain (Rosetta(DE3)/pET-IFN/pGB2) without IPTG induction; lane 3: a protein sample of a control strain (Rosetta(DE3)/pET-IFN/pGB2) with IPTG induction; lane 4: a protein sample of a strain (Rosetta(DE3)/pET-IFN/pGB-VHb) that produced *Vitreoscilla* hemoglobin after induction; lane 5: a protein sample of a strain (Rosetta(DE3)/pET-IFN/pGB-Trx) that produced thioredoxin after induction; and lane 6: a protein sample of a strain (Rosetta(DE3)/pET-IFN/pGB-TV1) that produced a fusion protein of thioredoxin and *Vitreoscilla* hemoglobin after induction;

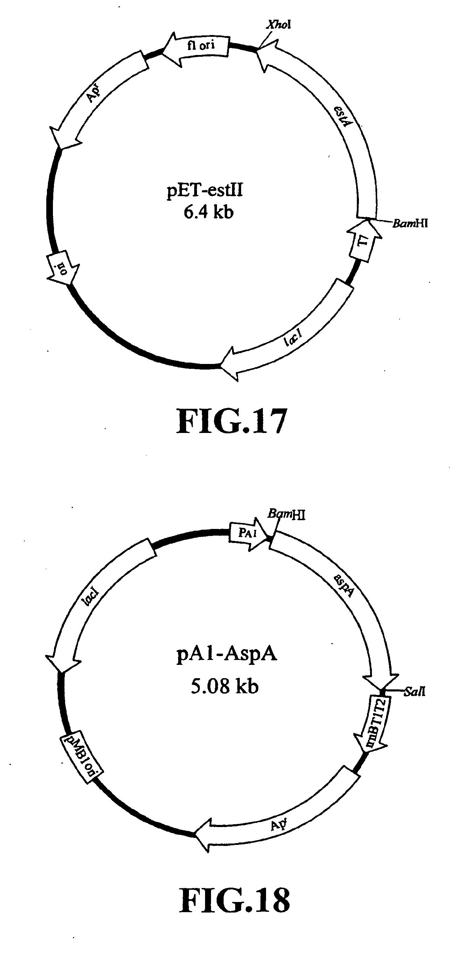

Figure 17 shows the construction of plasmid pET-estll, which contains: ori, the replication origin of pBR322; f1 ori, f1 replication origin; *estA*, esterase gene of *Pseudomonas citronellolis*; T7, T7 promoter; *BamH*I, the cutting site of restriction enzyme *BamH*I; *Xho*I*,* the cutting site of restriction enzyme *Xho*I; *lacl, lac* repressor protein gene; and Ap^{r}, ampicillin resistance gene;
Figure 18 shows the construction of plasmid pA1-AspA, which contains: pMB1 ori, replication origin of pMB1; *aspA*, aspartase gene; *BamH*I*,* the cutting site of restriction enzyme *BamH*I; *Sal*I, the cutting site of restriction enzyme *Sal*I; *rrnB*T1T2, *rrnB* gene transcriptional termination site; P_{A1}, T7 A1 promoter; *lacl, Lac* repressor protein gene; and Ap^{r}, ampicillin resistance gene;

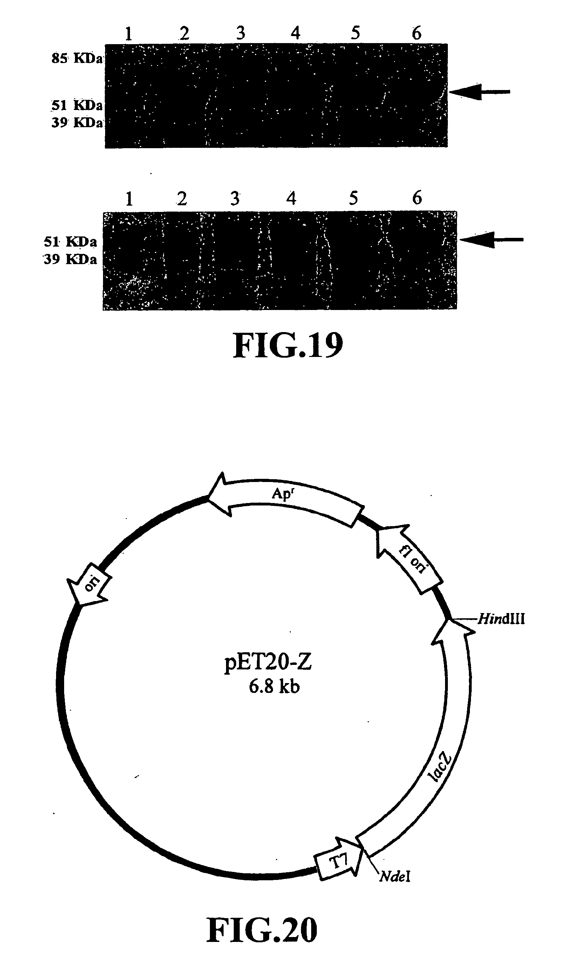

Figure 19 is a protein electrophoresis graph showing the production of proteins by recombinant strains that were induced to produce aspartase after fermentation culture for 7 hours (upper panel) and 20 hours (lower panel), wherein lane 1: protein standards (MBI Fermentas); lane 2: a protein sample of a control strain (VJS632/pA1-AspA/pGB2) that produced neither *Vitreoscilla* hemoglobin nor thioredoxin; lane 3: a protein sample of a strain (VJS632/pA1-AspA/pGB-VHb) that produced *Vitreoscilla* hemoglobin; lane 4: a protein sample of a strain (VJS632/pA1-AspA/pGB-Trx) that produced thioredoxin; lane 5: a protein sample of a strain (VJS632/pA1-AspA/pGB-TV2) that produced both *Vitreoscilla* hemoglobin and thioredoxin; lane 6: a protein sample of a strain (VJS632/pA1-AspA/pGB-TV1) that produced a fusion protein of thioredoxin and *Vitreoscilla* hemoglobin; and the arrows indicate the positions of aspartase; and
Figure 20 shows the construction of plasmid pET20-Z, which contains: ori, the replication origin of pBR322; f1 ori, f1 replication origin; *lacZ*, β-galactosidase structural gene; T7, T7 promoter; *Nde*I, the cutting site of restriction enzyme *Nde*I; *Hind*III, the cutting site of restriction enzyme *Hind*III; and Ap^{r}, ampicillin resistance gene.

In order to achieve the object of a high production of recombinant proteins, having considered the problems existing in the field of biotechnology, the applicants believe that the solution thereto can be sought from two aspects: one is to ensure the growth of the cells or to effectively increase the cell density of grown cells, and the other is to effectively increase the yield of recombinant proteins as produced by recombinant host cells.

Although the thioredoxin gene and the *Vitreoscilla* hemoglobin gene have been independently used to transform host cells for the purposes of producing recombinant proteins, up to the present, the applicants are unaware of any published article or patent publication which teaches or suggests the effect(s) of these two genes on transformed host cell(s) when used in combination.

The applicants found from research and study that when the thioredoxin gene and the *Vitreoscilla* hemoglobin gene are transferred into a host cell together, the transformed host cell thus formed can be imparted with the following effects: protecting the produced recombinant protein from being disintegrated; enhancing growth of the cell without eliciting adverse effect(s) caused by the metabolic burden due to the massive production of proteins in the cell; and increasing the yield of produced recombinant proteins. Judging from the obtained experimental results, this approach can indeed effectively solve the problems associated with the massive production of recombinant proteins mentioned hereinabove, and will be a very important contribution to the biotechnology industry.

Accordingly, this invention provides a method for enhancing the production of a selected gene product in a recombinant host cell, comprising the following steps of:
(a) cloning into a host cell a gene sequence encoding the selected gene product, a first nucleic acid sequence encoding thioredoxin, and a second nucleic acid sequence encoding Vitreoscilla hemoglobin, so as to form a recombinant host cell;
(b) cultivating a recombinant host cell formed from step (a) in a suitable medium, so as to allow the expression of said gene sequence; and
(c) harvesting the expressed gene product.

The terms "nucleic acid" and "nucleic acid sequence" as used herein refer to a deoxyribonucleotide or ribonucleotide sequence in single-stranded or double-stranded form, and comprise naturally occurring and known nucleotides or artificial chemical mimics. The term "nucleic acid" as used herein is interchangeable with the terms "gene," "cDNA," "mRNA," "oligo-nucleotide" and "polynucleotide" in use.

Unless otherwise indicated, a nucleic acid sequence, in addition to the specific sequences described herein, also covers its complementary sequence, and conservative analogs thereof, such as homologous sequences having degenerative codon substitutions. Specifically, degenerative codon substitutions may be produced by, for instance, a nucleotide residue substitution at the third position of one or more selected codons in a nucleic acid sequence with other nucleotide residue(s).

Techniques for manipulating nucleic acids, such as those for generating mutation in sequences, subcloning, labeling, probing, sequencing, hybridization and so forth, are described in detail in scientific publications and patent documents. See, for example, Sambrook J, Russell DW (2001) Molecular Cloning: a Laboratory Manual, 3rd ed. Cold Spring Harbor Laboratory Press, New York; Current Protocols in Molecular Biology, Ausubel ed., John Wiley & Sons, Inc., New York (1997); Laboratory Techniques in Biochemistry and Molecular Biology: Hybridization With Nucleic Acid Probes, Part I, Theory and Nucleic Acid Preparation, Tijssen ed., Elsevier, N.Y. (1993).

The terms "polypeptide," "peptide" and "protein" as used herein can be interchangeably used, and refer to a polymer formed of amino acid residues, wherein one or more amino acid residues are naturally occurring amino acids or artificial chemical mimics.

The terms "cell," "host cell," "transformed host cell" and "recombinant host cell" as used herein can be interchangeably used, and not only refer to specific individual cells but also include sub-cultured offsprings or potential offsprings thereof. Sub-cultured offsprings formed in subsequent generations may include specific genetic modifications due to mutation or environmental influences and, therefore, may factually not be fully identical to the parent cells from which the sub-cultured offsprings were derived. However, sub-cultured cells still fall within the coverage of the terms used herein.

Host cells that may be used in step (a) of the method according to this invention may be prokaryotic or eukaryotic cells, and may be non-transformed/transfected cells, or cells transformed/transfected with at least one other recombinant nucleic acid sequence.

Prokaryotic cells suitable for use in this invention include, but are not limited to, cells derived from: bacteria, e.g., *E. coli, Bacillus subtilis, Lactobacillus sp., Streptomyces sp.,* and *Salmonella typhi*; *Cyanobacteria*; *Actinomycetes, etc.*

Eukaryotic cells suitable for use in this invention include, for example, fungal cells, protozoan cells, plant cells, insect cells, animal cells, and human cells. Examples of suitable fungal cells are yeast cells, such as cells of *Saccharomyces cerevisiae* or *Pichia pastoris*. Suitable plant cells are those derived from gynosperms or angiosperms, preferably monocots and dicots, in particular crops, and are derived from the roots, shoots, leaves or meristems of these plants, and are cultured in the form of protoplasts or calli. Examples of suitable insect cells are *Drosophila* S2 cells, and Sf21 cells and Sf9 cells derived from *Spodoptera frugiperda*. Suitable animal cells may be cultured cells or cells *in vivo,* preferably derived from vertebrates, and more preferably mammals, and are derived from organs/tissues, such as kidney, liver, lung, ovary, breast, skin, skeleton and blood, of these animals. Representative examples of animal cells include CHO, COS, BHK, HEK-293, Hela, NIH3T3, VERO, MDCK, MOLT-4, Jurkat, K562, HepG2, etc.

Suitable culture media and culture conditions for host cells suitable for carrying out DNA recombination techniques are well known in the field of biotechnology. For instance, host cells may be cultured in a fermentation bioreactor, a shaking flask, a test tube, a microtiter plate, or a petri dish, and cultivation of the host cells may be conducted under conditions suitable for growth of said cells, including the culture temperature, the pH value of the culture medium, and the dissolved oxygen concentration of the culture.

In a preferred embodiment of this invention, step (a) is carried out by cloning the gene sequence, the first nucleic acid sequence and the second nucleic acid sequence into a vector expressible in the host cell, and transferring the recombinant vector thus formed into the host cell.

In another preferred embodiment of this invention, step (a) is carried out by cloning the gene sequence, the first nucleic acid sequence and the second nucleic acid sequence respectively into a vector expressible in the host cell, and transferring the respective recombinant vectors thus formed into the host cell together.

In a further preferred embodiment of this invention, step (a) is carried out by:
(i) constructing a first recombinant vector expressible in the host cell and including the first nucleic acid sequence and the second nucleic acid sequence;
(ii) constructing a second recombinant vector expressible in the host cell and including the gene sequence; and
(iii) transferring the first recombinant vector and the second recombinant vector into the host cell.

In a preferred embodiment of this invention, the first recombinant vector constructed in sub-step (i) further includes an inducible promoter sequence to control the expression of the first nucleic acid sequence and the second nucleic acid sequence.

In another preferred embodiment of this invention, the first recombinant vector constructed in sub-step (i) further includes a first inducible promoter sequence to control the expression of the first nucleic acid sequence, and a second inducible promoter sequence to control the expression of the second nucleic acid sequence, the first and second promoter sequences being different from each other.

The term "promoter sequence" as used herein refers to a DNA sequence, which is generally located upstream of a gene present in a DNA polymer, and provides a site for initiation of the transcription of said gene into mRNA. Promoter sequences suitable for use in this invention may be derived from viruses, bacteriophages, prokaryotic cells or eukaryotic cells, and may be a constitutive promoter or an inducible promoter.

According to this invention, the gene sequence encoding the selected gene product, the first nucleic acid encoding the thioredoxin, and the second nucleic acid encoding the Vitreoscilla hemoglobin may be independently and operatively linked to a promoter, particularly an inducible promoter.

The term "operatively linked" as used herein means that a first sequence is disposed sufficiently close to a second sequence such that the first sequence can influence the second sequence or regions under the control of the second sequence. For instance, a promoter sequence may be operatively linked to a gene sequence, and is normally located at the 5'-terminus of the gene sequence such that the expression of the gene sequence is under the control of the promoter sequence. In addition, a regulatory sequence may be operatively linked to a promoter sequence so as to enhance the ability of the promoter sequence in promoting transcription. In such case, the regulatory sequence is generally located at the 5'-terminus of the promoter sequence.

Promoter sequences suitable for use in this invention are preferably derived from any one of the following: viruses, bacterial cells, yeast cells, fungal cells, algal cells, plant cells, insect cells, animal cells, and human cells. For example, a promoter useful in bacterial cells includes, but is not limited to, *tac* promoter, T7 promoter, T7 A1 promoter, *lac* promoter, *trp* promoter, *trc* promoter, *araBAD* promoter, and λ*P_{R}P_{L}* promoter. A promoter useful in plant cells includes, *e*.*g*., 35S CaMV promoter, actin promoter, ubiquitin promoter, *etc.* Regulatory elements suitable for use in mammalian cells include CMV-HSV thymidine kinase promoters, SV40, RSV-promoters, CMV enhancers, or SV40 enhancers.

In a preferred embodiment of this invention, the first nucleic acid sequence encoding the thioredoxin and the second nucleic acid encoding the Vitreoscilla hemoglobin are cloned into a first vector, whereas the gene sequence encoding the selected gene product is cloned into a second vector. The first vector and the second vector respectively have inducible promoter sequences to control the expression of the first nucleic acid sequence and the second nucleic acid, and the expression of said gene sequence, respectively.

In a preferred embodiment of this invention, expression of the first nucleic acid and the second nucleic acid that are cloned into the first recombinant vector is controlled by the same promoter, such as *tac* promoter, T7 promoter, T7 A1 promoter, *lac* promoter, *trp* promoter, *trc* promoter, *araBAD* promoter, or λ*P_{R}P_{L}*promoter, and preferably a *tac* promoter.

In a preferred embodiment of this invention, the first nucleic acid sequence and the second nucleic acid sequence are constructed in frame as to encode a fusion protein formed of thioredoxin and Vitreoscilla hemoglobin.

In another preferred embodiment, the first nucleic acid sequence and the second nucleic acid are cloned into the first recombinant vector, and these two nucleic acid sequences are controlled by different promoters for expression, the promoters being independently selected from the group consisting of: *tac* promoter, T7 promoter, T7 A1 promoter, *lac* promoter, *trp* promoter, *trc* promoter, *araBAD* promoter, and *P_{R}P_{L}* promoter. In a more preferred embodiment, the first recombinant vector includes a *tac* promoter to control the expression of the first nucleic acid sequence, and a T7 A1 promoter to control the expression of the second nucleic acid sequence.

Vectors suitable for use in this invention include those commonly used in genetic engineering technology, such as bacteriophages, plasmids, cosmids, viruses, or retroviruses.

Vectors suitable for use in this invention may include other expression control elements, such as a transcription starting site, a transcription termination site, a ribosome binding site, a RNA splicing site, a polyadenylation site, a translation termination site, *etc.* Vectors suitable for use in this invention may further include additional regulatory elements, such as transcription/translation enhancer sequences, and at least a marker gene or reporter gene allowing for the screening of the vectors under suitable conditions. Marker genes suitable for use in this invention include, for instance, dihydrofolate reductase gene and G418 or neomycin resistance gene useful in eukaryotic cell cultures, and ampicillin, streptomycin, tetracycline or kanamycin resistance gene useful in *E. coli* and other bacterial cultures. Vectors suitable for use in this invention may further include a nucleic acid sequence encoding a secretion signal. These sequences are well known to those skilled in the art.

The term "expression vector" as used herein refers to any recombinant expression system capable of expressing the first nucleic acid sequence, the second nucleic acid sequence, and the gene sequence according to this invention in any host cell *in vitro* or *in vivo,* constitutively or inducibly. The expression vector may be an expression system in linear or circular form, and covers expression systems that remain episomal or integrate into the host cell genome. The expression system may or may not have the ability to self-replicate, and it may drive only transient expression in a host cell.

According to this invention, the term "transformation" can be used interchangeably with the term "transfection" when such term is used to refer to the introduction of an exogenous nucleic acid molecule into a selected host cell. According to techniques known in the art, a nucleic acid molecule (*e*.*g*., a recombinant DNA construct or a recombinant vector) can be introduced into a selected host cell by various techniques, such as calcium phosphate- or calcium chloride-mediated transfection, electroporation, microinjection, particle bombardment, liposome-mediated transfection, transfection using bacterial bacteriaphages, transduction using retroviruses or other viruses (such as vaccinia virus or baculovirus of insect cells), protoplast fusion, *Agrobacterium*-mediated transformation, or other methods.

Depending on the vector and host cell system used, the recombinant gene product (protein) produced according to this invention may either remain within the recombinant cell, be secreted into the culture medium, be secreted into periplasm, or be retained on the outer surface of a cell membrane. The recombinant gene product (protein) produced by the method of this invention can be purified by using a variety of standard protein purification techniques, including, but not limited to, affinity chromatography, ion exchange chromatography, gel filtration, electrophoresis, reverse phase chromatography, chromatofocusing and the like. The recombinant gene product (protein) produced by the method of this invention is preferably recovered in "substantially pure" form. As used herein, the term "substantially pure" refers to a purity of a purified protein that allows for the effective use of said purified protein as a commercial product.

According to this invention, the first nucleic acid sequence is derived from a thioredoxin encoding gene of any of the following cells: bacterial cells, yeast cells, fungal cells, algal cells, plant cells, insect cells, animal cells, and human cells. In a preferred embodiment of this invention, the first nucleic acid sequence is derived from the thioredoxin gene (*trxA*) of *E. coli.*

According to this invention, the second nucleic acid sequence is the hemoglobin gene (*vgb*) of *Vitreoscilla sp.*

The method according to this invention can be used to produce a homologous polypeptide or a heterologous polypeptide. In a preferred embodiment of this invention, the gene product to be produced by the method of this invention is selected from the group consisting of: enzymes, e.g., β-galactosidase, esterase and aspartase; therapeutic polypeptides, e.g., interferons, interleukins, animal or human hormones; antigenic determinants; and antibodies. Preferably, the gene product is an antigenic determinant derived from any of the following: pathogenic viruses, bacteria or other microorganisms, parasites or tumor cells.

This invention also provides a nucleic acid construct, which comprises a first nucleic acid sequence encoding thioredoxin, and a second nucleic acid sequence encoding Vitreoscilla hemoglobin.

In a preferred embodiment of this invention, the nucleic acid construct may further comprise an inducible promoter sequence to control the expression of the first nucleic acid sequence and the second nucleic acid sequence. Preferably, the promoter sequence is derived from any of the following: viruses, bacterial cells, yeast cells, fungal cells, algal cells, plant cells, insect cells, animal cells, and human cells. According to this invention, the promoter sequence may be selected from the group consisting of *tac* promoter, T7 promoter, T7 A1 promoter, *lac* promoter, *trp* promoter, *trc* promoter, *araBAD* promoter and λ*P_{R}P_{L}* promoter, and is preferably a *tac* promoter.

According to a preferred embodiment of this invention, the first nucleic acid sequence and the second nucleic acid sequence are connected so as to encode a fusion protein formed of thioredoxin and Vitreoscilla hemoglobin.

In a preferred embodiment of this invention, the nucleic acid construct further comprises a first inducible promoter sequence to control the expression of the first nucleic acid sequence, and a second inducible promoter sequence to control the expression of the second nucleic acid sequence, the first and second promoter sequences being different from each other.

Preferably, the first promoter sequence and the second promoter sequence are independently derived from any of the following: viruses; bacterial cells, yeast cells, fungal cells, algal cells, plant cells, insect cells, animal cells and human cells. According to this invention, the first promoter sequence and the second promoter sequence are independently selected from the group consisting of *tac* promoter, T7 promoter, T7 A1 promoter, *trp* promoter, *lac* promoter, *trc* promoter, *araBAD* promoter, and λ*P_{R}P_{L}* promoter. According to a preferred embodiment of this invention, the first promoter sequence is *tac* promoter, and the second promoter sequence is T7 A1 promoter.

This invention also provides a vector comprising the nucleic acid construct described hereinabove. According to this invention, the vector may further include at least one of the following: a marker gene, a reporter gene, an antibiotic-resistance gene, an enhancer sequence, a gene encoding a selected gene product, a polyadenylation site, and a regulatory sequence.

This invention also provides a recombinant host cell capable of expressing a selected gene product, the cell comprising a gene sequence encoding the selected gene product, a first nucleic acid sequence encoding thioredoxin, and a second nucleic acid sequence encoding Vitreoscilla hemoglobin.

In a preferred embodiment of this invention, the gene sequence, the first nucleic acid sequence and the second nucleic acid sequence together are carried by a vector expressible in the host cell. In another preferred embodiment of this invention, the gene sequence, the first nucleic acid sequence and the second nucleic acid sequence are independently carried by a vector expressible in the host cell. In still another preferred embodiment of this invention, the first nucleic acid sequence and the second nucleic acid sequence are both carried by a first vector expressible in the host cell, and the gene sequence is carried by a second vector expressible in the host cell.

According to this invention, the first vector may further comprise an inducible promoter sequence to control the expression of the first nucleic acid sequence and the second nucleic acid sequence. The promoter sequence may be derived from any of the following: viruses, bacterial cells, yeast cells, fungal cells, algal cells, plant cells, insect cells, animal cells, and human cells. Preferably, the promoter sequence is selected from the group consisting of *tac* promoter, T7 promoter, T7 A1 promoter, *lac* promoter, *trp* promoter, *trc* promoter, *araBAD* promoter and λ*P_{R}P_{L}* promoter, and is preferably *tac* promoter.

In a preferred embodiment of this invention, the first nucleic acid sequence and the second nucleic acid sequence harbored in the recombinant host cell are connected so as to encode a fusion protein formed of thioredoxin and Vitreoscilla hemoglobin.

In a preferred embodiment of this invention, the first vector harbored in the recombinant host cell further comprises a first inducible promoter sequence to control the expression of the first nucleic acid sequence, and a second inducible promoter sequence to control the expression of the second nucleic acid sequence, the first and second promoter sequences being different from each other.

According to this invention, the first promoter sequence and the second promoter are independently derived from any of the following: viruses, bacterial cells, yeast cells, fungal cells, algal cells, plant cells, insect cells, animal cells, and human cells. Preferably, the first promoter sequence and the second promoter sequence are independently selected from the group consisting of *tac* promoter, T7 promoter, T7 A1 promoter, *lac* promoter, *trp* promoter, *trc* promoter, *araBAD* promoter, and λ*P_{R}P_{L}* promoter. In a preferred embodiment of this invention, the first promoter sequence is *tac* promoter, and the second promoter is T7 A1 promoter.

According to this invention, the first vector that harbored in the recombinant host cell further comprises a marker gene, a reporter gene, an antibiotic-resistance gene, an enhancer sequence, a polyadenylation site, or a regulatory sequence.

In a preferred embodiment of this invention, the first nucleic acid sequence and the second nucleic acid sequence are incorporated into the genomic DNA of the host cell.

The recombinant host cell according to this invention may produce a selected homologous or heterologous polypeptide. In a preferred embodiment of this invention, the recombinant host cell produces a gene product selected from the group consisting of: enzymes, such as β-galactosidase, esterase, and aspartase; therapeutic polypeptides, such as interferons, interleukins, animal or human hormones; antigenic determinants or antibodies. Preferably, the gene product is an antigenic determinant derived from any of the following: pathogenic viruses, bacteria or other microorganisms, parasites, and tumor cells.

According to this invention, the recombinant host cell is selected from the group consisting of bacterial cells, yeast cells, fungal cells, plant cells, insect cells, animal cells, and human cells. In a preferred embodiment of this invention, the recombinant host cell is an *E. coli* cell.

This invention will be further described by way of the following examples. However, it should be understood that the following examples are intended for the purpose of illustration only and should not be construed as limiting the invention in practice.

### Examples

### General experimental methods and materials:

Concerning the experimental methods and relevant techniques for DNA cloning as employed in this invention, such as DNA cleavage reaction by restriction enzymes, DNA ligation with T4 DNA ligase, polymerase chain reaction (PCR), agarose gel electrophoresis, sodium dodecyl sulfate-polyacrylamide gel electrophoresis (SDS-PAGE), Western blotting and plasmid transformation, etc., reference is made to a textbook widely known in the art: Sambrook J, Russell DW (2001) Molecular Cloning: a Laboratory Manual, 3rd ed. Cold Spring Harbor Laboratory Press, New York. These techniques can be readily performed by those skilled in the art based on their professional knowledge and experience. For instance, in the examples described hereinbelow, calcium chloride-treated competent cells were used to conduct plasmid transformation. In addition, cell density was measured using a spectrophotometer (V530, Jasco) at a wavelength of 550 nm, and the obtained absorbance was recorded as OD₅₅₀. Protein concentration analysis was conducted using a protein assay reagent (BioRad Co.), and an image analyzer (GAS9000, UVltec) was used to determine the contents of proteins on an electrophored gel.

Chromosomes, plasmids and DNA fragments were respectively purified using the following commercially available purification kits: Wizard@ Genomic DNA Purification Kit (Promega Co.), QIAprep® Spin Miniprep kit (Qiagen Co.) and NucleoSpin® Nucleic Acid Purification Kit (Clontech Co.).

All of the restriction enzymes, T4 DNA ligase, and Pfu DNA polymerase were purchased from New England Biolabs. Primers used in the polymerase chain reaction (PCR) were synthesized by Mission Biotech Co. (Taipei, Taiwan, R.O.C.). Primary antibodies used to detect *Vitreoscilla* hemoglobin were prepared by Abnova Taiwan Co. (Taipei, Taiwan, R.O.C.). The primary antibodies and the secondary antibodies (*i*.*e*. horseradish peroxidase-conjugated goat anti-rabbit IgG) used to detect thioredoxin, as well as other chemicals, were purchased from Sigma Chemical Co.

### Example 1. Construction of expression plasmids containing Vitreoscilla hemoglobin structural gene (vgb) and thioredoxin structural gene (trxA)

### Experimental Materials:

An *E. coli* strain XL1-Blue (Stratagene Co.) was used as intermediate cells in the DNA cloning process. The bacterial cells were cultured at 37 °C in Luria-Bertani (LB) medium (Miller, J.H. (1972), Experiments in Molecular Genetics, Cold Spring Harbor Laboratory, Cold Spring Harbor, NY). Thereafter, transformed *E. coli* cells were cultured in a medium supplemented with antibiotic(s), in which the used amount of antibiotic is 15 µg/mL for streptomycin, and 50 µg/mL for ampicillin.

*Vitreoscilla* sp. (Murray strain no. 389) was kindly afforded by Dr. Webster (Department of Biology, Illinois Institute of Technology, Chicago, USA). The bacterial cells were cultured at 30°C in a medium containing 1.5% yeast extract, 1.5% peptone, and 0.02% sodium acetate.

### A. Construction of a recombinant plasmid pGB-VHb carrying a Vitreoscilla hemoglobin structural gene (vgb)

A recombinant plasmid pGB-VHb carrying a *Vitreoscilla* hemoglobin structural gene (*vgb*) under the control of a T7 A1 promoter is constructed as follows:

The following two primers were synthesized based on the nucleotide sequence of *Vitreoscilla* hemoglobin structural gene (*vgb*) (Khosla C, Bailey JE. (1988), Mol Gen Genet., 214:158-161):
Forward primer
   5'-aagggatccatgttagaccagcaaacc-3' (SEQ. ID. NO.: 1)
Reverse primer
   5'-atagtcaacccaagttttggcaacagc-3' (SEQ. ID NO.: 2)

The two primers described above were designed to have a cutting site of restriction enzyme *BamH*I or *Sal*I (see the underlined portions), respectively.

*Vitreoscilla* chromosomes were purified using Wizard@ Genomic DNA Purification Kit, and were used as a template in a PCR reaction using the aforesaid two primers, so that a DNA fragment (0.55 kb) containing *Vitreoscilla* hemoglobin structural gene *(vgb)* was amplified. Thereafter, the amplified DNA fragment was purified using NucleoSpin® Nucleic Acid Purification Kit, followed by cleavage using restriction enzymes *BamH*I/*Sal*I. The resultant cleavage product was then incorporated into a plasmid pUHE21-2 cleaved by *BamH*I/*Sal*I (as shown in Figure 1, this plasmid was kindly afforded by Dr. Bujard, Heidelbrg University, Germany, and comprises an ampicillin resistance gene, pMB1 replication origin, T7 A1 promoter, the cutting site of restriction enzyme *Sca*I, and multiple cloning sites), so that a plasmid called "pUHE-VHb" was obtained.

Thereafter, a DNA fragment carrying the ribosome binding site and *Vitreoscilla* hemoglobin structural gene *(vgb)* was cleaved from the plasmid pUHE-VHb using restriction enzymes *EcoR*I/*Hind*III, followed by ligating to a plasmid pA199A-2 cleaved by *EcoR*I/*Hind*III (as shown in Figure 2, this plasmid comprises an ampicillin resistance gene, pMB1 replication origin, *lacl* gene, T7 A1 promoter and multiple cloning sites)(Y-P. Chao et al. (2003), Biotechnol Prog. 19: 1076-1080), so that a plasmid called "pA1A2-VHb" was obtained.

Finally, a DNA fragment containing *lacl* gene, T7 A1 promoter and *Vitreoscilla* hemoglobin structural gene *(vgb)* was cleaved from the plasmid pA1A2-VHb using restriction enzymes *Nru*I/*Hind*III, followed by incorporation into a plasmid pGB2 cleaved by *Sma*I/*Hind*III (as shown in Figure 3, this plasmid comprises a streptomycin resistance gene, pSC101 replication origin and multiple cloning sites)(G. Churchward et al. (1984), Gene, 31:165-171), so that a plasmid called "pGB-VHb" and having a structure as shown in Figure 4 was obtained.

The plasmid pGB-VHb was subjected to a sequencing analysis conducted by Mission Biotech Co. (Taipei, Taiwan, R.O.C.), and the obtained results proved that the nucleic acid sequence of T7 A1 promoter and the nucleic acid sequence of *Vitreoscilla* hemoglobin structural gene (*vgb*) are included in the plasmid pGB-VHb.

### B. Construction of a recombinant plasmid pGB-Trx carrying a thioredoxin structural gene (trxA)

A plasmid pGB-Trx carrying a thioredoxin structural gene (*trx*A) under the control of a *tac* promoter is constructed as follows:

The following two primers were synthesized based on the nucleotide sequence of thioredoxin structural gene (*trxA*) (C. J. Lim et al. (1985), J Bacteriol., 163:311-316):
Forward primer
   5'-ccgaattcaaccacacctatggtgtatgc-3' (SEQ. ID. NO.: 3)
Reverse primer
   5'-ggggatccgctgcaaggcgattaag-3' (SEQ. ID. NO.: 4)

The two primers described above were designed to have a cutting site of restriction enzyme *EcoR*I or *BamH*I (see the underlined portions), respectively.

Plasmid pTrx (Invitrogen Co.) was purified using QIAprep® Spin Miniprep kit, and was used as a template in a PCR reaction using the aforesaid two primers, so that a DNA fragment (0.58 kb) containing thioredoxin structural gene (*trxA*) was amplified.

Thereafter, the amplified DNA fragment was purified using the NucleoSpin® Nucleic Acid Purification Kit, followed by cleavage with restriction enzymes *EcoR*I/*BamH*I. The resultant cleaved product was then incorporated into a plasmid pJF118EH cleaved by *EcoR*I/*BamH*I (as shown in Figure 5, this plasmid comprises an ampicillin resistance gene, pBR322 replication origin, *lacl*^{q} gene, *tac* promoter, and multiple cloning sites)(J.P. Furste et al. (1986), Gene, 48:119-131), so that a plasmid called "pJF-Trx" was obtained.

Finally, a DNA fragment containing *lacl*^{q} gene, *tac* promoter and thioredoxin structural gene (*trxA*) was cleaved from the plasmid pJF-Trx using restriction enzymes *Nru*I/*Hind*III, followed by incorporation into a plasmid pGB2 cleaved by *Sma*I/*Hind*III, so that a plasmid called "pGB-Trx" and having a structure as shown in Figure 6 was obtained.

The plasmid pGB-Trx was subjected to a sequencing analysis conducted by Mission Biotech Co. (Taipei, Taiwan, R.O.C.), and the obtained results proved that the nucleotide sequence of *tac* promoter and the nucleotide sequence of thioredoxin structural gene (*trxA*) are included in the plasmid pGB-Trx.

### C. Construction of a recombinant plasmid pGB-TV1 carrying a thioredoxin structural gene (trxA) fused with a Vitreoscilla hemoglobin structural gene (vgb)

A plasmid pGB-TV1 carrying a thioredoxin structural gene (*trxA*) fused with a *Vitreoscilla* hemoglobin structural gene (*vgb*) under the control of a *tac* promoter is constructed as follows: A DNA fragment containing *Vitreoscilla* hemoglobin structural gene (*vgb*) was cleaved from the plasmid pUHE-VHb obtained in Procedure A described above using restriction enzymes *BamH*I/*Hind*III, followed by incorporation into a plasmid pKF3 (Takara Shuzo Co.) cleaved by *BamH*I/*Hind*III, so that a plasmid called "pKF-VHb" was obtained.

Subsequently, a DNA fragment containing *Vitreoscilla* hemoglobin structural gene (*vgb*) was cleaved from the plasmid pKF-VHb using restriction enzymes *BamH*I/*SacI,* followed by incorporation into a plasmid pBluescript II-SK (Stratagene Co.) cleaved by *BamH*I/*SacI,* so that a plasmid called "pBlue-VHb" was obtained.

Thereafter, a DNA fragment containing a complete *Vitreoscilla* hemoglobin structural gene (*vgb*) was cleaved from the plasmid pBlue-VHb using restriction enzymes *Sma*I/*Pst*I, followed by incorporation into a plasmid pJF-TrxFus (which, as shown in Figure 7, has an ampicillin resistance gene, pBR322 replication origin, *lacl*^{q} gene, *tac* promoter, the cutting site of restriction enzyme *Nru*I, and multiple cloning sites, and a thioredoxin structural gene without a stop codon located downstream of the *tac* promoter)(Y-P. Chao et al. (2000), Appl. Microbiol. Biotechnol., 54: 348-353) cleaved by *Sma*I/*Pst*I, so that a plasmid called "pJF-Trx/VHb" was obtained.

Finally, a DNA fragment containing the *lacl*^{q} gene, *tac* promoter, and thioredoxin structural gene (*trxA*) fused with *Vitreoscilla* hemoglobin structural gene (*vgb*) was cleaved from the plasmid pJF-Trx/VHb using restriction enzymes *Nru*I/*Hind*III, followed by incorporation into a plasmid pGB2 cleaved by *Sma*I/*Hind*III, so that a plasmid called "pGB-TV1" and having a structure as shown in Figure 8 was obtained.

The plasmid pGB-TV1 was subjected to a sequencing analysis conducted by Mission Biotech Co. (Taipei, Taiwan, R.O.C.), and the obtained results proved that the nucleic acid sequence of *tac* promoter and a nucleic acid sequence constituted of thioredoxin structural gene (*trxA*) fused with *Vitreoscilla* hemoglobin structural gene *(vgb)* are included in the plasmid pGB-TV1.

### D. Construction of a recombinant plasmid pGB-TV2 carrying a thioredoxin structural gene (trxA) and a Vitreoscilla hemoglobin structural gene (vgb)

A plasmid pGB-TV2 carrying a *Vitreoscilla* hemoglobin structural gene (*vgb*) and a thioredoxin structural gene (*trxA*) respectively under the control of a T7 A1 promoter and a *tac* promoter is constructed as follows:

A DNA fragment containing a T7 A1 promoter and a complete *Vitreoscilla* hemoglobin structural gene (*vgb*) was cleaved from the plasmid pUHE-VHb obtained in Procedure A described above using restriction enzymes *Sca*I/*Hind*III*,* and a DNA fragment containing *lacl*^{q} gene, *tac* promoter and a complete thioredoxin structural gene (*trxA*) was cleaved from the plasmid pJF-Trx obtained in Procedure B described above using restriction enzymes *Nru*I/*BamH*I. Subsequently, these two DNA fragments were incorporated into a plasmid pGB2 cleaved by *BamH*I/*Hind*III*,* so that a plasmid called "pGB-TV2" and having a structure as shown in Figure 9 was obtained.

The plasmid pGB-TV2 was subjected to a sequencing analysis conducted by Mission Biotech Co. (Taipei, Taiwan, R.O.C.), and the obtained results proved that the plasmid thus obtained has a vector structure as shown in Figure 9, wherein *Vitreoscilla* hemoglobin structural gene (*vgb*) is located downstream of the T7 A1 promoter, and thioredoxin structural gene (*trxA*) is located downstream of the *tac* promoter.

### Example 2. Production of thioredoxin, Vitreoscilla hemoglobin, and a fusion protein of thioredoxin and Vitreoscilla hemoglobin

According to the plasmid transformation method described in the preceding section of "General experimental methods," the bacterial cells of an *E. coli* strain BL21 (DE3)(Novagen Co.) were transformed with the four recombinant plasmids pGB-VHb, pGB-Trx, pGB-TV1 and pGB-TV2 obtained in Example 1, respectively, so that four recombinant *E. coli* strains BL21(DE3)/pGB-VHb, BL21(DE3)/pGB-Trx, BL21(DE3)/pGB-TV1 and BL21(DE3)/ pGB-TV2 were respectively obtained.

Colonies of the recombinant strains described above were individually selected from solid agar plates, and were inoculated into a 5 mL LB culture medium containing 15 µg/mL streptomycin, followed by cultivation at 37°C overnight. Thereafter, the overnight-cultured cells of different recombinant strains were inoculated into a 250-mL flask containing 20 mL LB culture medium (containing 15 µg/mL streptomycin), respectively. When an initial cell density of 0.05 (OD₅₅₀) was reached, the freshly inoculated bacterial culture was cultivated in a shaking incubator set at a constant temperature of 37°C and a rotation speed of 250 rpm. After a cell density of about 0.3 (OD₅₅₀) was reached, different concentrations of IPTG were added into individual bacterial cultures of different recombinant strains so as to induce the recombinant *E. coli* cells cultivated therein to produce recombinant protein products.

After addition of the inducing agent for 4 hours, the bacterial cells were collected by centrifugation and broken with French Press (Thermo Spectronic, NY), followed by separation using a freeze centrifuge. Thereafter, the recovered supernatants were subjected to a protein analysis using Protein Assay Reagent (BioRad Co.) so as to determine the protein concentrations thereof, and to an immunodetection by Western blotting so as to determine the yields of recombinant proteins as produced.

Western blotting was conducted according to the descriptions set forth in the preceding section of "General experimental methods." The collected samples (each containing 20 µg protein therein) were loaded onto a 15% polyacrylamide gel in sequence for electrophoresis. After electrophoresis, the separated proteins on the gel were transferred to a nitrocellulose membrane using Electrophoretic Transfer Cell (BioRad) at a constant current of 300 mA for three hours. The thus transferred nitrocellulose membrane was subjected to a binding treatment using a primary antibody at room temperature for three hours, followed by rinsing twice using a rinsing buffer containing 1% gelatin. Subsequently, the nitrocellulose membrane was immersed in a solution containing a second antibody with shaking at room temperature for 1 hour, followed by rinsing twice with a rinse buffer containing 1% gelatin. Thereafter, the nitrocellulose membrane was immersed in a developing solution containing 9 mg 4-chloro-1-naphthol until colored bands appeared. The nitrocellulose membrane was then taken out and washed with deionized water to stop the color-developing reaction.

Figure 10 is a Western blot showing the immunodetection of *Vitreoscilla* hemoglobin produced by an IPTG-induced recombinant *E. coli* strain BL21(DE3)/pGB-VHb using a primary antibody against *Vitreoscilla* hemoglobin, wherein lane 1: protein standards (ProSieve® Color Protein Marker, Cambrex BioScience); lane 2: a protein sample of the strain without IPTG induction; lane 3: a protein sample of the strain induced with 30 µM IPTG; lane 4: a protein sample of the strain induced with 100 µM IPTG; lane 5: a protein sample of the strain induced with 500 µM IPTG; and the arrow indicates the position of *Vitreoscilla* hemoglobin.

Figure 11 is a Western blot showing the immunodetection of thioredoxin produced by an IPTG-induced recombinant *E. coli* strain BL21(DE3)/pGB-Trx using a primary antibody against thioredoxin, wherein lane 1: protein standards (ProSieve® Color Protein Marker, Cambrex BioScience); lane 2: a protein sample of the strain without IPTG induction; lane 3: a protein sample of the strain induced with 30 µM IPTG; lane 4: a protein sample of the strain induced with 100 µM IPTG; lane 5: a protein sample of the strain induced with 500 µM IPTG; and the arrow indicates the position of thioredoxin.

Figure 12 is a Western blot showing the immunodetection of a fusion protein of thioredoxin and *Vitreoscilla* hemoglobin produced by an IPTG-induced recombinant *E. coli* strain BL21(DE3)/pGB-TV1 using a primary antibody against thioredoxin, wherein lane 1: protein standards (ProSieve® Color Protein Marker, Cambrex BioScience); lane 2: a protein sample of the strain without IPTG induction; lane 3: a protein sample of the strain induced with 30 µM IPTG; lane 4: a protein sample of the strain induced with 100 µM IPTG; lane 5: a protein sample of the strain induced with 500 µM IPTG; and the arrow indicates the position of the fusion protein of thioredoxin and *Vitreoscilla* hemoglobin.

Figure 13 is a Western blot showing the immunodetection of thioredoxin produced by an IPTG-induced recombinant *E. coli* strain BL21(DE3)/pGB-TV2 using a primary antibody against thioredoxin, wherein lane 1: protein standards (ProSieve® Color Protein Marker, Cambrex BioScience); lane 2: a protein sample of the strain without IPTG induction; lane 3: a protein sample of the strain induced with 30 µM IPTG ; lane 4: a protein sample of the strain induced with 100 µM IPTG; lane 5: a protein sample of the strain induced with 500 µM IPTG; and the arrow indicates the position of thioredoxin.

Figure 14 is a Western blot showing the immunodetection of *Vitreoscilla* hemoglobin produced by an IPTG-induced recombinant *E. coli* strain BL21(DE3)/pGB-TV2 using a primary antibody against *Vitreoscilla* hemoglobin, wherein lane 1: protein standards (ProSieve® Color Protein Marker, Cambrex BioScience); lane 2: a protein sample of the strain without IPTG induction; lane 3: a protein sample of the strain induced with 30 µM IPTG; lane 4: a protein sample of the strain induced with 100 µM IPTG; lane 5: a protein sample of the strain induced with 500 µM IPTG; and the arrow indicates the position of *Vitreoscilla* hemoglobin.

It can be seen from Figures 10-14 that production of recombinant proteins is not detectable in recombinant strains without IPTG induction. After addition of IPTG for induction, the recombinant strains could be induced to produce recombinant proteins, and the amounts of the proteins produced will increase with an increase in the amount of the inducing agent. These results show that recombinant *E. coli* strains transformed according to this invention are capable of producing thioredoxin, *Vitreoscilla* hemoglobin, or a fusion protein of thioredoxin and *Vitreoscilla* hemoglobin. In addition, the yields of recombinant proteins as produced by these recombinant *E. coli* strains can be controlled by using different concentrations of IPTG.

### Example 3. Use of transformed E. coli cells in the production of a heterologous protein - human interferon α2

A plasmid pET-IFN carrying a human interferon α2 structural gene under the control of a T7 promoter is constructed as follows:

The following two primers were synthesized based on the nucleotide sequence of human interferon α2 structural gene (E. Austruy et al. (1998), Cancer Gene Ther., 5:247-256):
Forward primer
   5'-tgctctcatatgttg atctgcctcaaac-3'(SEQ. ID. NO.:5)
Reverse primer
   5'-cgtgctcgag ttattattccttacttcttaaac-3' (SEQ. ID. NO.: 6)

The two primers described above were designed to have a cutting site of restriction enzyme *Nde*I or *Xho*I (see the underlined portions), respectively.

A plasmid pIFN2A (kindly afforded by Professor Hsu Ju An of the National Health Institute) derived from plasmid pET22b(+)(Novagen Co.) and carrying a human interferon α2 gene was purified using QIAprep® Spin Miniprep kit, and was subsequently used as a template in a PCR reaction using the aforesaid two primers, so that a DNA fragment containing human interferon α2 structural gene (INFα2) was amplified.

Thereafter, the amplified DNA fragment was purified with NucleoSpin® Nucleic Acid Purification Kit, followed by cleavage with restriction enzymes *Nde*I*lXho*I. The resultant cleaved product was incorporated into a plasmid pET22b (Novagen Co.), so that a plasmid called "pET-IFN" and having a structure as shown in Figure 15 was obtained.

According to the plasmid transformation method described in the preceding section of "General experimental methods," the bacterial cells of an *E. coli* strain Rosetta(DE3)(Novagen Co.) were respectively transformed with plasmids pGB2, pGB-VHb, pGB-Trx and pGB-TV1, together with the plasmid pET-IFN, so that four recombinant *E. coli* strains, *i.e.* Rosetta(DE3)/pET-IFN/pGB2, Rosetta(DE3)/pET-IFN/pGB-VHb, Rosetta(DE3)/pET-IFN/pGB-Trx and Rosetta(DE3)/pET-IFN/pGB-TV1, were respectively obtained. These recombinant strains were cultivated substantially according to the procedures described in Example 2, except that the culture media were added with both streptomycin and ampicillin in amounts as described in the preceding section of "General experimental methods."

When a cell density of about 0.3 (OD₅₅₀) was reached, the freshly inoculated bacterial cultures were added with 500 µM IPTG so as to induce the recombinant *E. coli* cells cultivated therein to produce recombinant protein products. When the bacterial cells grew to a state approximating the early stationary growth phase (6 hours after addition of the inducing agent), the bacterial cells were collected by centrifugation, and the amounts of recombinant proteins produced thereby were determined by protein electrophoresis (*i*.*e*., sodium dodecyl sulfate-polyacrylamide gel electrophoresis, SDS-PAGE).

SDS-PAGE was conducted according to the descriptions set forth in the preceding section of "General experimental methods." The collected cells were boiled in boiling water for 3 minutes, followed by separation with a freeze centrifuge. The recovered supernatants were subjected to a protein analysis using Protein Assay Reagent (BioRad Co.) so as to determine the protein concentrations thereof. Samples of the collected supernatant (containing 20 µg protein therein) were loaded onto a 15% polyacrylamide gel in sequence for electrophoresis, followed by detection using an image analyzer (GAS9000, UVltec) so as to quantitatively determine the contents of proteins present on the electrophored gel. The obtained experimental results are shown in Table 1.

**Table 1. The growth results of recombinant strains induced to produce human interferon α2**

| Strain Rosefta(DE3) Plasmid | IPTG-induction (µM) | Final cell density (OD₅₅₀) |
|---|---|---|
| pET-IFN/pGB2 | 0 | 4.00 |
| | 500 | 1.54(1)* |
| PET-IFN/pGB-VHb | 500 | 1.36(0.88) |
| pET-IFN/pGB-Trx | 500 | 2.63(1.7) |
| PET-IFN/pGB-TV1 | 500 | 2.65(1.7) |

| | | |
|---|---|---|
| *: The numbers within the parentheses respectively stand for the ratios of the cell densities of the recombinant strains versus that of the control strain. The relative cell density is calculated by dividing the cell density of each induced recombinant strain listed in the table by the cell density of the induced control strain Rosetta(DE3)/pET-IFN/pGB2. | | |

At the end of fermentation, the cell density of the control strain Rosetta(DE3)/pET-IFN/pGB2 without being induced to produce human interferon α2 could reach 4.0 (OD₅₅₀). However, when IPTG was added to induce the production of human interferon α2, the growth of the control strain, *i*.*e*. the strain Rosetta(DE3)/pET-IFN/pGB2 which produced neither *Vitreoscilla* hemoglobin nor thioredoxin, was immediately suppressed. This phenomenon of cell growth suppression also occurred in the *Vitreoscilla* hemoglobin-producing strain Rosetta(DE3)/pET-IFN/pGB-VHb upon being induced to produce human interferon α2. Finally, the cell densities of these two strains reached 1.54 (OD₅₅₀) and 1.36 (OD₅₅₀), respectively.

However, after induction with IPTG, both the thioredoxin-producing strain Rosetta(DE3)/pET-IFN/pGB-Trx and the strain Rosetta(DE3)/pET-IFN/pGB-TV1 which produced a fusion protein of thioredoxin and *Vitreoscilla* hemoglobin were shown to have an increased growth by 70%, and their final cell densities reached approximately 2.65 (OD₅₅₀), as compared to the control strain Rosetta(DE3)/pET-IFN/pGB2 which was induced to produce human interferon α2.

In addition, the protein electrophoresis graph of Figure 16 shows that, as compared to the control strain Rosetta(DE3)/pET-IFN/pGB2 which produced neither *Vitreoscilla* hemoglobin nor thioredoxin, the yield of human interferon α2 produced by the *Vitreoscilla* hemoglobin-producing strain Rosetta(DE3)/pET-IFN/pGB-VHb was decreased by 53%, and the yield of human interferon α2 produced by the thioredoxin-producing strain (Rosetta(DE3)/pET-IFN/pGB-Trx) was likewise decreased by 35%. On the other hand, for the strain Rosetta(DE3)/pET-IFN/pGB-TV1 which produced a fusion protein of thioredoxin and *Vitreoscilla* hemoglobin, the yield of human interferon α2 produced thereby was equivalent to that of the control strain. As compared to the control strain, the amount of human interferon α2 produced by the thioredoxin-producing strain Rosetta(DE3)/pET-IFN/pGB-Trx was decreased. The observed less amount of human interferon α2 produced by this strain may account for the better cell growth thereof.

In conclusion, as compared to the control strain (Rosetta(DE3)/pET-IFN/pGB2) without IPTG induction to produce human interferon α2, the growth of induced control strain was severely suppressed. However, as compared to induced control strains, the strain (Rosetta(DE3)/pET-IFN/pGB-TV1) which produced a fusion protein of thioredoxin and *Vitreoscilla* hemoglobin could produce an equivalent amount of human interferon α2, and the cell density thereof was increased by 70%. This indicates that a vector carrying a thioredoxin gene fused with a *Vitreoscilla* hemoglobin gene can impart a host cell transformed thereby with an ability to relieve toxic effect(s) arising from overproduction of recombinant protein product(s), e.g. a human interferon.

### Example 4. Use of transformed E. coli cells in the production of a heterologous protein - Pseudomonas citronellolis esterase

In our previous studies, a plasmid pET-estll (Figure 17) containing a T7 promoter to control the expression of *Pseudomonas citronellolis* esterase gene was prepared (Y.P. Chao et al. (2003), Res. Microbiol., 154:521-526).

According to the plasmid transformation method described in the preceding section of "General experimental methods," the bacterial cells of an *E. coli* strain BL21(DE3) were respectively transformed with plasmids pGB2, pGB-VHb, pGB-Trx, pGB-TV1, and pGB-TV2, together with the plasmid pET-estll, so that five recombinant *E. coli* strains, *i*.*e*. BL21 (DE3)/pET-estll/pGB2, BL21 (DE3)/pET-estll/pGB-VHb, BL21 (DE3)/pET-estll/pGB-Trx, BL21 (DE3)/pET-estll/pGB-TV1 and BL21 (DE3)/pET-estll/pGB-TV2, were respectively obtained.

Cultivation of these recombinant strains was conducted according to the procedures described in Example 3. When a cell density of about 0.3 (OD₅₅₀) was reached, the freshly inoculated bacterial cultures were added wtih 300 µM IPTG so as to induce the recombinant *E. coli* cells cultivated therein to produce recombinant protein products. 6 hours after addition of the inducing agent (*i*.*e*., 9 hours of fermentation), the bacterial cells were collected by centrifugation and then subjected to detection in terms of the activity of esterase produced thereby.

Detection of the activity of *Pseudomonas citronellolis* esterase was conducted based on the method reported in our previous paper (Y.P. Chao et al. (2003), Res. Microbiol., 154:521-526). Essentially, the collected bacterial cells were broken using a high-pressure homogenizer (French Press, USA), followed by separation using a freeze centrifuge. The recovered supernatants were subjected to a protein analysis using Protein Assay Reagent (BioRad Co.) so as to determine the protein concentrations thereof. To a 400 µL reaction solution (containing 100 mM sodium phosphate buffer solution (pH 7.4), 0.1% arabic gum and 0.4% Triton X-100) was added 6 µL cell supernatant and 10 mM of para-nitrophenol acetate. Reaction was carried out at room temperature, and the yield of reaction product was recorded with time by detecting the absorbance of the reaction mixture at a wavelength of 410 nm, based upon which the specific activity (U/mg) of this enzyme was calculated. The enzyme activity unit (U) was defined as the amount of enzyme that catalyzed the release of 1 µmole of product per minute. The obtained experimental results are shown in Table 2.

**Table 2. The growth and enzyme production results of recombinant strains induced to produce Pseudomonas citronellolis esterase**

| Strain BL21(DE3) Plasmid | IPTG-induction (µM) | Final cell density (OD₅₅₀) | Enzyme specific activity (U/mg) |
|---|---|---|---|
| PET-estII/pGB2 | 0 | 5.2 | - |
| | 300 | 1.7(1)* | 15.5(1)* |
| pET-estII/pGB-VHb | 300 | 1.7(1) | 11.5(0.7) |
| PET-estII/pGB-Trx | 300 | 4.2(2.5) | 6.0(0.4) |
| pET-estII/pGB-TV1 | 300 | 3.7(2.2) | 15.0(1) |
| pET-estII/pGB-TV2 | 300 | 3.5(2.1) | 15.5(1) |

| | | | |
|---|---|---|---|
| *: The numbers within the parentheses respectively stand for the ratio of the cell density of each recombinant strain versus that of the control strain, and the ratio of the produced enzyme specific activity of each recombinant strain versus that of the control strain. Relative cell density and relative enzyme specific activity were calculated by dividing the cell density and the enzyme specific activity of each induced recombinant strain listed in the table by the cell density and the enzyme specific activity of the induced control strain BL21(DE3)/pET-estII/pGB2, respectively. | | | |

It can be seen from this experiment that at the end of fermentation, the cell density of the control strain BL21(DE3)/pET-estll/pGB2 without being induced to produce esterase could reach 5.2 (OD₅₅₀). However, after induction with IPTG to induce the production of esterase, the growth of the control strain, *i*.*e*. the strain BL21(DE3)/pET-estll/pGB2 which produced neither *Vitreoscilla* hemoglobin nor thioredoxin, was immediately suppressed. This phenomenon of cell growth suppression also occurred in the *Vitreoscilla* hemoglobin-producing strain BL21(DE3)/pET-estll/pGB-VHb upon being induced to produce esterase. Finally, the cell densities of these two strains reached only 1.7 (OD₅₅₀), respectively.

However, after induction with IPTG, the thioredoxin-producing strain BL21(DE3)/pET-estll/pGB-Trx, the strain BL21(DE3)/pET-estll/pGB-TV2 which produced both *Vitreoscilla* hemoglobin and thioredoxin, and the strain BL21(DE3)/pET-estll/pGB-TV1 which produced a fusion protein of thioredoxin and *Vitreoscilla* hemoglobin, were all shown to have an increased growth by 110-150%, and their final cell densities could reach 4.2 (OD₅₅₀), 3.5 (OD₅₅₀) and 3.7 (OD₅₅₀), respectively, as compared to the control strain BL21(DE3)/pET-estll/pGB2 which was induced to produce esterase.

It can be seen from the enzyme production results shown in Table 2 that after IPTG induction, the control strain BL21(DE3)/pET-estII/pGB2 which produced neither *Vitreoscilla* hemoglobin nor thioredoxin could achieve an esterase specific activity up to 15.5 U/mg, whereas the strain BL21(DE3)/pET-estII/pGB-VHb which produced *Vitreoscilla* hemoglobin achieved an esterase specific activity of 11.5 U/mg. As for the strain BL21(DE3)/pET-estII/pGB-TV2 which produced *Vitreoscilla* hemoglobin and thioredoxin, or the strain BL21(DE3)/pET-estII/pGB-TV1 which produced a fusion protein of thioredoxin and *Vitreoscilla* hemoglobin, the esterase specific activities thereof reached 15.5 U/mg and 15 U/mg, respectively, both values being comparable to that of the control strain BL21(DE3)/pET-estll/pGB2 after IPTG induction.

As compared to the control strain, the esterase production of the thioredoxin-producing strain BL21 (DE3)/pET-estll/pGB-Trx was decreased by 60% (enzyme specific activity reached 6 U/mg only). The observed less amount of esterase produced by this strain may account for the better cell growth thereof.

In conclusion, as compared to the control strain BL21(DE3)/pET-estII/pGB2 without being induced to produce esterase, the growth of the induced control strain was severely suppressed. However, as compared to the induced control strain, the strain BL21(DE3)/pET-estll/pGB-TV2 which produced both *Vitreoscilla* hemoglobin and thioredoxin, or the strain BL21(DE3)/pET-estll/pGB-TV1 which produced a fusion protein of thioredoxin and *Vitreoscilla* hemoglobin could produce equivalent amounts of esterase, and the cell density thereof was increased by more than 110%. This indicates that production of both *Vitreoscilla* hemoglobin and thioredoxin, or production of a fusion protein of thioredoxin and *Vitreoscilla* hemoglobin could impart transformed recombinant host cells with the ability to relieve toxic effect(s) arising from overproduction of recombinant protein products, e.g. *Pseudomonas citronellolis* esterase.

### Example 5. Use of transformed E. coli cells in the production of a homologous protein - aspartase

A plasmid pA1-AspA carrying an *E. coli* aspartase gene under the control of a T7A1 promoter is constructed as follows:

The following two primers were synthesized based on the nucleotide sequence of aspartase gene (S.A. Woods et al. (1986), Biochem. J., 237:547-557):
Forward primer
   5'-cacaggatccacaacattcgtatcgaag-3' (SEQ. ID. NO.: 7)
Reverse primer
   5'-acgagtcgacttcgctttcatcagtatag-3' (SEQ. ID. NO.: 8)

The two primers described above were designed to have a cutting site of restriction enzyme *BamH*I or *Sal*I (see the underlined portions), respectively.

Chromosomes of the bacterial cells of a wild-type *E. coli* strain VJS632 (kindly afforded by Dr. Stewart, University of California, Davis, CA, USA) were purified using Wizard@ Genomic DNA Purification Kit, and were subsequently used as a template in a PCR reaction using the aforesaid two primers, so that a DNA fragment containing aspartase gene (*aspA*) was amplified.

Thereafter, the amplified DNA fragment was purified using NucleoSpin® Nucleic Acid Purification Kit, followed by cleavage with the restriction enzymes *BamH*I*lSal*I. The resultant cleaved product was incorporated into a plasmid pA199A-2, so that a plasmid called "pA1-AspA" and having a structure as shown in Figure 18 was obtained.

According to the plasmid transformation method described in the preceding section of "General experimental methods," the bacterial cells of an *E. coli* strain VJS632 were respectively transformed with plasmids pGB2, pGB-VHb, pGB-Trx, pGB-TV1 and pGB-TV2, together with the plasmid pA1-AspA, so that five recombinant *E. coli* strains, *i*.*e*. VJS632/pA1-AspA/pGB2, VJS632/pA1-AspA/pGB-VHb, VJS632/pA1-AspA/pGB-Trx, VJS632/pA1-AspA/pGB-TV1 and VJS632/pA1-AspA/pGB-TV2, were respectively obtained.

Cultivation of the recombinant strains was conducted according to the method described in Example 3, with the exception of using a culture medium containing the following components: Na₂HPO₄ (6 g/L), KH₂PO₄ (3 g/L), NaCl (0.5 g/L), NH₄Cl (1 g/L), MgSO₄ • 7H₂O (1 mM), CaCl₂ (0.1 mM), glucose (0.4%), as well as streptomycin (10 µg/mL) and ampicillin (15 µg/mL). When a cell density of about 0.3 (OD₅₅₀) was reached, the freshly inoculated bacterial cultures were added with 1000 µM IPTG so as to induce the recombinant *E. coli* cells cultivated therein to produce recombinant protein products. During the course of cultivation, the bacterial cultures were sampled with time for measurement of cell density. The bacterial cells were collected by centrifugation after cultivation for 7 hours and 20 hours, respectively, and were subsequently subjected to detection in terms of yield of recombinant protein and enzyme activity.

Protein electrophoresis (SDS-PAGE) was conducted according to the method described in Example 3, and supernatant samples (containing 20 µg protein therein) of the collected bacterial cell samples were loaded in sequence onto a 10% polyacrylamide gel for electrophoresis. The activity of *E. coli* aspartase was determined based on the method described in the applicant's previous paper (Y.-P. Chao et al. (2000), Enzyme Microb Technol, 27:19-25). Essentially, 0.05 mg of protein was added into 1 mL reaction solution containing 100 mM aspartic acid, 100 mM Tris buffer (pH 8.4) and 5 mM MgSO₄. After reaction at room temperature for 10 minutes, the reaction product (fumaric acid) was analyzed by HPLC under the following conditions: column, Rezex ROA column (Phenomenex Co.); mobile phase, 0.005 N sulfuric acid; flow rate, 0.5 mL/min; and detection of absorbance was conducted at a wavelength of 210 nm. The enzyme specific activity (U/mg) was calculated based on the absorbance detected, and the enzyme activity unit (U) was defined as the amount of enzyme that catalyzed the release of 1 µmole of product per minute. The obtained experimental results are shown in Table 3.

**Table 3. Production of enzyme by recombinant strains which were induced to produce aspartase at different fermentation times**

| Strain VJS632 Plasmid | IPTG-induction (mM) | Enzyme specific activity (U/mg) | |
|---|---|---|---|
| | | Fermentation for 7 hours | Fermentation for 20 hours |
| pA1-AspA/pGB2 | 1 | 20.0 | 3.7(0.19)* |
| pA1-AspA/pGB-VHb | 1 | 21.1 | 6.1 (0.29) |
| PA1-AspA/pGB-Trx | 1 | 19.9 | 12.5(0.63) |
| pA1-AspA/pGB-TV1 | 1 | 20.9 | 21.1 (1) |
| pA1-AspA/pGB-TV2 | 1 | 20.1 | 20.0(1) |

| | | | |
|---|---|---|---|
| *: The numbers within the parentheses respectively stand for the ratios of the enzyme specific activities obtained after 20-hour fermentation versus those obtained after 7-hour fermentation, of the recombinant strains. The relative enzyme specific activity of each recombinant strain was calculated by dividing the enzyme specific activity obtained after 20-hour fermentation by that obtained after 7-hour fermentation. | | | |

Referring to the upper panel of Figure 19, after 7-hour fermentation, all of the recombinant strains which were induced to produce aspartase could generate a considerable amount of aspartase, with an enzyme specific activity of up to about 20 U/mg (see Table 3). Referring to the lower panel of Figure 19, after 20-hour fermentation, the enzyme activities of aspartase produced by the control strain VJS632/pA1-AspA/pGB2 which produced neither *Vitreoscilla* hemoglobin nor thioredoxin, the strain VJS632/pA1-AspA/pGB-VHb which produced *Vitreoscilla* hemoglobin, and the strain VJS632/pA1-AspA/pGB-Trx which produced thioredoxin reached only 3.7 U/mg, 6.1 U/mg and 12.5 U/mg, with a drop of 81%, 71% and 37%, respectively. It is also observed from the protein electrophoresis results shown in Figure 19 that the amounts of aspartase produced by the aforesaid strains were significantly decreased.

In contrast, for the strain VJS632/pA1-AspA/pGB-TV2 which produced both *Vitreoscilla* hemoglobin and thioredoxin, or the strain VJS632/pA1-AspA/pGB-TV1 which produced a fusion protein of thioredoxin and *Vitreoscilla* hemoglobin, the amounts of aspartase produced thereby were not adversely influenced (see the lower panel of Figure 19), with enzyme specific activities being maintained at about 20 U/mg (Table 3).

In conclusion, the production of *Vitreoscilla* hemoglobin and thioredoxin or the production of a fusion protein of thioredoxin and *Vitreoscilla* hemoglobin could impart transformed recombinant host cells with the ability to protect and stabilize recombinant protein products (e.g., aspartase) produced thereby, thereby ensuring that the recombinant protein products will not be subjected to adverse disintegrating action(s) of proteases present in the recombinant host cells.

### Example 6. Use of transformed E. coli cells in the production of a homologous protein - β-galactosidase

A plasmid pET20-Z carrying a β-galactosidase gene under the control of a T7 promoter is constructed as follows:

The following two primers were synthesized based on the nucleotide sequence of β-galactosidase gene (A. Kalnins et al. (1983), EMBO J. 2; 593-597):
Forward primer
   5'-tatgcatatgaggatccattcactggccgtc-3' (SEQ. ID. NO.: 17)
Reverse primer
   5'-cggaagcttttatttttgacaccagacc-3' (SEQ. ID. NO.:18)

The two primers described above were designed to have a cutting site of restriction enzyme *Nde*I or *Xho*I (see the underlined portions), respectively.

Chromosomes of the bacterial cells of an *E. coli* strain BL21(DE3) were purified using Wizard@ Genomic DNA Purification Kit, and were subsequently used as a template in a PCR reaction using the aforesaid two primers, so that a DNA fragment containing β-galactosidase gene (*lacZ*) was amplified.

Thereafter, the amplified DNA fragment was purified using NucleoSpin® Nucleic Acid Purification. Kit, followed by cleavage with restriction enzymes *Ndel*I/*Xho*I. The resultant cleaved product was incorporated into a plasmid pET22b pre-treated with the same restriction enzymes, so that a plasmid called "pET20-Z" and having a structure as shown in Figure 20 was obtained.

According to the plasmid transformation method described in the preceding section of "General experimental methods," the bacterial cells of an *E. coli* strain BL21(DE3) were respectively transformed with plasmids pGB2, pGB-VHb, pGB-Trx, pGB-TV1 and pGB-TV2, together with the plasmid pET20-Z, so that five recombinant *E. coli* strains, *i*.*e*. BL21(DE3)/pET20-Z/pGB2, BL21 (DE3)/pET20-Z/pGB-VHb, BL21 (DE3)/pET20-Z/pGB-Trx, BL21 (DE3)/pET20-Z/pGB-TV1 and BL21 (DE3)/pET20-Z/pGB-TV2, were respectively obtained.

Cultivation of the recombinant strains was conducted according to the method described in Example 5.

When a cell density of about 0.3 (OD₅₅₀) was reached, the freshly inoculated bacterial cultures were added with 300 µM IPTG so as to induce the recombinant *E. coli* cells cultivated therein to produce recombinant protein products. During the course of cultivation, the bacterial cultures were sampled with time for measurement of cell density. The bacterial cells were collected by centrifugation after cultivation for 11 hours, and were subsequently subjected to detection in terms of the enzyme activity of β-galactosidase produced thereby.

Detection of the activity of *E. coli* β-galactosidase was conducted according to the method described in Miller JH (1972) Experiments in Molecular Genetics. Cold Spring Harbor, NY: Cold Spring Harbor Laboratory.

Essentially, aliquots (0.1 mL) of bacterial cultures were sampled with time and independently admixed with 0.9 mL Z buffer solution (16.1 g/L Na₂HPO₄ • 7H₂O, 5.5 g/L NaH₂PO₄ • H₂O, 0.75 g/L KCI, 0.246 g/L MgSO₄ • 7H₂O, 2.7 mL β-mercaptoethanol), so as to maintain a final volume of 1 mL. Thereafter, 10 µL toluene was added. The bacterial cells were broken using a votexer, followed by separation using a freeze centrifuge so as to collect supernatants.

To each of the collected supernatants was added a 0.2 mL reaction solution containing *o*-nitrophenyl-β-D-thiogalactoside (4 mg/mL). Reaction was conducted at room temperature for 5 minutes, followed by the addition of 0.5 mL Na₂CO₃ (1 M) to stop reaction. Production of the reaction product was measured by detecting absorbance at a wavelength of 420 nm. The specific enzyme activity unit of β-galactosidase was defined as Miller unit. The obtained experimental results are shown in Table 4.

**Table 4. The growth and enzyme production results of recombinant strains induced to produce β-galactosidase**

| Strain BL21(DE3) Plasmid | IPTG-induction (µM) | Final cell density (OD₅₅₀) | Enzyme specific activity (Miller unit) |
|---|---|---|---|
| pET20-Z/pGB2 | 0 | 3.2 | - |
| | 300 | 0.7 (1)* | 56300 (1)* |
| pET20-Z/pGB-VHb | 300 | 0.9 (1.3) | 51700 (0.9) |
| pET20-Z/pGB-Trx | 300 | 2.3 (3.3) | 40400 (0.7) |
| pET20-Z/pGB-TV1 | 300 | 2.5 (3.6) | 83200 (1.5) |
| pET20-Z/pGB-TV2 | 300 | 1.7 (2.4) | 97000 (1.7) |

| | | | |
|---|---|---|---|
| *: The numbers within the parentheses respectively stand for the ratio of the cell density of each recombinant strain versus that of the control strain, and the ratio of the produced enzyme specific activity of each recombinant strain versus that of the control strain. Relative cell density and relative enzyme specific activity were calculated by dividing the cell density and the enzyme specific activity of each induced recombinant strain listed in the table by the cell density and the enzyme specific activity of the induced control strain BL21(DE3)/pET20-Z/pGB2, respectively. | | | |

At the end of fermentation, the control strain (*i*.*e*., the strain BL21(DE3)/pET20-Z/pGB2 which produced neither *Vitreoscilla* hemoglobin nor thioredoxin) without being induced to produce β-galactosidase could reach a cell density of 3.2 (OD₅₅₀). However, upon addition of IPTG to induce the production of β-galactosidase, the growths of the control strain and the strain BL21 (DE3)/pET20-Z/pGB-VHb which produced *Vitreoscilla* hemoglobin were severely suppressed immediately, and their final cell densities reached only 0.7-0.9 (OD₅₅₀) at the end of cultivation. As compared to the induced control strain, the strain BL21(DE3)/pET20-Z/pGB-Trx which produced thioredoxin, and the strain (BL21(DE3)/pET20-Z/pGB-TV1) which produced a fusion protein of thioredoxin and *Vitreoscilla* hemoglobin were shown to have a final cell density of 2.3-2.5 (OD₅₅₀) at the end of fermentation, with an increase in cell density by 230-260%. The cell density of the strain BL21 (DE3)/pET20-Z/pGB-TV2 which produced both *Vitreoscilla* hemoglobin and thioredoxin could also reach 1.7 (OD₅₅₀), with an increase in cell density by 140%.

On the other hand, as observed from the protein production results in terms of enzyme specific activity shown in Table 4, after IPTG induction, the control strain BL21 (DE3)/pET20-Z/pGB2 could produce β-galactosidase of about 56,000 Miller units, and the strain BL21(DE3)/pET20-Z/pGB-VHb which produced *Vitreoscilla* hemoglobin could produce β-galactosidase of about 51,000 Miller units as well. However, the strain BL21(DE3)/pET20-Z/pGB-TV2 which produced both *Vitreoscilla* hemoglobin and thioredoxin could produce β-galactosidase of about 97,000 Miller units, and the strain BL21(DE3)/pET20-Z/pGB-TV1 which produced a fusion protein of thioredoxin and *Vitreoscilla* hemoglobin could produce β-galactosidase of about 83,000 Miller units, with an increase in β-galactosidase production by 50-70%, as compared to the induced control strain (BL21 (DE3)/pET20-Z/pGB2).

In contrast, the strain BL21(DE3)/pET20-Z/pGB-Trx which produced thioredoxin could produce β-galactosidase of only 40,000 Miller units, with a drop in β-galactosidase production by about 30%, as compared to the induced control strain (BL21(DE3)/pET20-Z/pGB2). The observed less amount of β-galactosidase produced by this strain may account for the better cell growth thereof.

In conclusion, regardless of whether both *Vitreoscilla* hemoglobin and thioredoxin are produced or a fusion protein of thioredoxin and *Vitreoscilla* hemoglobin is produced, the transformed host cells can be imparted with the ability to increase β-galactosidase production and to result in better growth. According to a report by H. Dong et al. (1995), J. Bacteriol. 177:1497-1504, overproduction of β-galactosidase will lead to disintegration of ribosomes of *E. coli* cells, thus causing cell growth to be severely suppressed. Therefore, it is suggested that transformed strains, which can produce both *Vitreoscilla* hemoglobin and thioredoxin, or a fusion protein of thioredoxin and *Vitreoscilla* hemoglobin, may have the ability to retard disintegration of cell ribosomes due to an overproduction of protein therein.

In case of conflict, the present description, including definitions, will prevail.

## Claims

1. A method for enhancing the production of a selected gene product in a recombinant host cell, comprising:
(a) cloning into a host cell a gene sequence encoding the selected gene product, a first nucleic acid sequence encoding thioredoxin, and a second nucleic acid sequence encoding *Vitreoscilla* hemoglobin, so as to form a recombinant host cell;
(b) cultivating a recombinant host cell formed from step (a) in a suitable medium, so as to allow the expression of said gene sequence ; and
(c) harvesting the expressed gene product.

2. A method according to claim 1, wherein step (a) is conducted by cloning said gene sequence, said first nucleic acid sequence and said second nucleic acid sequence together in a vector expressible in said host cell, and transferring the thus formed recombinant vector into the host cell.

3. A method according to claim 1, wherein step (a) is conducted by independently cloning said gene sequence, said first nucleic acid sequence and said second nucleic acid sequence into a vector expressible in said host cell, and transferring the thus formed recombinant vectors into the host cell.

4. A method according to claim 1, wherein step (a) is conducted by:
(i) constructing a first recombinant vector expressible in the host cell and including the first nucleic acid sequence and the second nucleic acid sequence;
(ii) constructing a second recombinant vector expressible in the host cell and including the gene sequence ; and
(iii) transferring the first recombinant vector and the second recombinant vector into the host cell.

5. A method according to claim 4, wherein said first recombinant vector constructed from the sub-step (i) further includes an inducible promoter sequence to control the expression of said first nucleic acid sequence and said second nucleic acid sequence.

6. A method according to claim 5, wherein said promoter sequence included in said first recombinant vector constructed from the sub-step (i) is derived from any of the following: viruses, bacterial cells, yeast cells, fungal cells, algal cells, plant cells, insect cells, animal cells, and human cells.

7. A method according to claim 6, wherein the promoter sequence included in said first recombinant vector constructed from the sub-step (i) is selected from the group consisting of tac promoter, T7 promoter, T7 A1 promoter, *lac* promoter, *trp* promoter, *trc* promoter, *araBAD* promoter, and *P_{R}P_{L}* promoter.

8. A method according to claim 7, wherein the promoter sequence included in said first recombinant vector constructed from the sub-step (i) is *tac* promoter.

9. A method according to claim 4, wherein, in said first recombinant vector constructed from the sub-step (i), said first nucleic acid sequence and said second nucleic acid sequence are connected to encode a fusion protein formed of thioredoxin and hemoglobin.

10. A method according to claim 4, wherein said first recombinant vector constructed from the sub-step (i) further includes an inducible first promoter sequence to control the expression of said first nucleic acid sequence, and an inducible second promoter sequence to control the expression of said second nucleic acid sequence, said first promoter sequence and said second promoter sequence being different from each other.

11. A method according to claim 10, wherein, in said first recombinant vector constructed from the sub-step (i), said first promoter sequence and said second promoter sequence are independently derived from any of the following: viruses, bacterial cells, yeast cells, fungal cells, algal cells, plant cells, insect cells, animal cells, and human cells.

12. A method according to claim 11, wherein, in said first recombinant vector constructed from the sub-step (i), said first promoter sequence and said second promoter sequence are independently selected from the group consisting of *tac* promoter, T7 promoter, T7 A1 promoter, *lac* promoter, *trp* promoter, *trc* promoter, *araBAD* promoter, and *P_{R}P_{L}* promoter.

13. A method according to claim 11, wherein, in said first recombinant vector constructed from the sub-step (i), said first promoter sequence is tac promoter and said second promoter is T7 A1 promoter.

14. A method according to claim 4, wherein said first recombinant vector constructed from the sub-step (i) further includes at least one of the following: a marker gene, a reporter gene, an antibiotic-resistance gene, an enhancer sequence, a polyadenylation site, and a regulatory sequence.

15. A method according to claim 4, wherein said first nucleic acid sequence used in step (a) is derived from the thioredoxin gene of any cell of the following: bacterial cells, yeast cells, fungal cells, algal cells, plant cells, insect cells, animal cells, and human cells.

16. A method according to claim 15, wherein said first nucleic acid sequence used in step (a) is derived from the thioredoxin gene (*trxA*) of *E. coli.*

17. A method according to claim 16, wherein said second nucleic acid sequence used in step (a) is derived from the hemoglobin gene (*vgb*) of *Vitreoscilla sp.*

18. A method according to claim 1, wherein said selected gene product is a homologous polypeptide or a heterologous polypeptide.

19. A method according to claim 18, wherein said selected gene product is an enzyme, a therapeutic polypeptide, an antigenic determinant, or an antibody.

20. A method according to claim 19, wherein said selected gene product is selected from the group consisting of interferon, β-galactosidase, esterase and aspartase.

21. A method according to claim 1, wherein the host cell used in step (a) is selected from the group consisting of bacterial cells, yeast cells, fungal cells, insect cells, plant cells, animal cells, and human cells.

22. A method according to claim 21, wherein the host cell used in step (a) is an *E. coli* cell.

23. A nucleic acid construct, comprising a first nucleic acid sequence encoding thioredoxin and a second nucleic acid sequence encoding *Vitreoscilla* hemoglobin.

24. A nucleic acid construct according to claim 23, further comprising an inducible promoter sequence to control the expression of said first nucleic acid sequence and said second nucleic acid sequence.

25. A nucleic acid construct according to claim 24, wherein said promoter sequence is derived from any of the following: viruses, bacterial cells, yeast cells, fungal cells, algal cells, plant cells, insect cells, animal cells, and human cells.

26. A nucleic acid construct according to claim 25, wherein said promoter sequence is selected from the group consisting of *tac* promoter, T7 promoter, T7 A1 promoter, lac promoter, *trp* promoter, *trc* promoter, *araBAD* promoter, and *P_{R}P_{L}* promoter.

27. A nucleic acid construct according to claim 25, wherein said promoter sequence is tac promoter.

28. A nucleic acid construct according to claim 24, wherein said first nucleic acid sequence and said second nucleic acid sequence are connected to encode a fusion protein formed of thioredoxin and hemoglobin.

29. A nucleic acid construct according to claim 23, further comprising an inducible first promoter sequence to control the expression of said first nucleic acid sequence, and an inducible second promoter sequence to control the expression of said second nucleic acid sequence, said first promoter sequence and said second promoter sequence being different from each other.

30. A nucleic acid construct according to claim 29, wherein said first promoter sequence and said second promoter sequence are independently derived from any of the following: viruses, bacterial cells, yeast cells, fungal cells, algal cells, plant cells, insect cells, animal cells, and human cells.

31. A nucleic acid construct according to claim 30, wherein said first promoter sequence and said second promoter sequence are independently selected from the group consisting of *tac* promoter, T7 promoter, T7 A1 promoter, lac promoter, *trp* promoter, *trc* promoter, *araBAD* promoter, and *P_{R}P_{L}* promoter.

32. A nucleic acid construct according to claim 30, wherein said first promoter sequence is *tac* promoter and said second promoter sequence is T7 A1 promoter.

33. A nucleic acid construct according to claim 23, wherein said first nucleic acid sequence is derived from the thioredoxin gene of any cell of the following: bacterial cells, yeast cells, fungal cells, algal cells, plant cells, insect cells, animal cells, and human cells.

34. A nucleic acid construct according to claim 33, wherein said first nucleic acid sequence is derived from the thioredoxin gene (*trxA*) of *E. coli.*

35. A vector comprising a nucleic acid construct according to any one of Claims 24-34.

36. A vector according to claim 35, further comprising at least one of the following: a marker gene, a reporter gene, an antibiotic-resistance gene, an enhancer sequence, a gene encoding a selected gene product, a polyadenylation site, and a regulatory sequence.

37. An isolated recombinant host cell capable of expressing a selected gene product, comprising a gene sequence encoding said selected gene product, a first nucleic acid sequence encoding thioredoxin, and a second nucleic acid sequence encoding *Vitreoscilla* hemoglobin.

38. An isolated recombinant host cell according to claim 37, wherein said gene sequence, said first nucleic acid sequence and said second nucleic acid sequence together are carried in a vector expressible in said host cell.

39. An isolated recombinant host cell according to claim 37, wherein said gene sequence, said first nucleic acid sequence and said second nucleic acid sequence are independently carried in a vector expressible in said host cell.

40. An isolated recombinant host cell according to claim 37, wherein said first nucleic acid sequence and said second nucleic acid sequence are carried in a first vector expressible in said host cell, and said gene sequence is carried in a second vector expressible in said host cell.

41. An isolated recombinant host cell according to claim 40, wherein said first vector further comprises an inducible promoter sequence to control the expression of said first nucleic acid sequence and said second nucleic acid sequence.

42. An isolated recombinant host cell according to claim 41, wherein said promoter sequence is derived from any of the following: viruses, bacterial cells, yeast cells, fungal cells, algal cells, plant cells, insect cells, animal cells, and human cells.

43. An isolated recombinant host cell according to claim 42, wherein said promoter sequence is selected from the group consisting of *tac* promoter, T7 promoter, T7 A1 promoter, *lac* promoter, *trp* promoter, *trc* promoter, *araBAD* promoter, and *P_{R}P_{L}* promoter.

44. An isolated recombinant host cell according to claim 43, wherein said promoter sequence is tac promoter.

45. An isolated recombinant host cell according to claim 40, wherein said first nucleic acid sequence and said second nucleic acid sequence are connected to encode a fusion protein formed of thioredoxin and hemoglobin.

46. An isolated recombinant host cell according to claim 40, wherein said first vector further comprises an inducible first promoter sequence to control the expression of said first nucleic acid sequence, and an inducible second promoter sequence to control the expression of said second nucleic acid sequence, said first promoter sequence and said second promoter sequence being different from each other.

47. An isolated recombinant host cell according to claim 46, wherein said first promoter sequence and said second promoter are independently derived from any of the following: viruses, bacterial cells, yeast cells, fungal cells, algal cells, plant cells, insect cells, animal cells, and human cells.

48. An isolated recombinant host cell according to claim 47, wherein said first promoter sequence and said second promoter sequence are independently selected from the group consisting of *tac* promoter, T7 promoter, T7 A1 promoter, *lac* promoter, *trp* promoter, *trc* promoter, *araBAD* promoter, and *P_{R}P_{L}* promoter.

49. An isolated recombinant host cell according to claim 48, wherein said first promoter sequence is tac promoter and said second promoter is T7 A1 promoter.

50. An isolated recombinant host cell according to claim 37, wherein said first nucleic acid sequence is derived from the thioredoxin gene of any cell of the following: bacterial cells, yeast cells, fungal cells, algal cells, plant cells, insect cells, animal cells, and human cells.

51. An isolated recombinant host cell according to claim 50, wherein said first nucleic acid sequence is derived from the thioredoxin gene (*trxA*) of *E. coli.*

52. An isolated recombinant host cell according to claim 40, wherein said first vector further comprises at least one of a marker gene, a reporter gene, an antibiotic-resistance gene, an enhancer sequence, a polyadenylation site, and a regulatory sequence.

53. An isolated recombinant host cell according to claim 37, wherein said first nucleic acid sequence and said second nucleic acid sequence are incorporated into the genomic DNA of said host cell.

54. An isolated recombinant host cell according to claim 37, wherein said selected gene product is a homologous polypeptide or a heterologous polypeptide.

55. An isolated recombinant host cell according to claim 54, wherein said selected gene product is an enzyme, a therapeutic polypeptide, an antigenic determinant or an antibody.

56. An isolated recombinant host cell according to claim 55, wherein said selected gene product is selected from the group consisting of interferon, β-galactosidase, esterase, and aspartase.

57. An isolated recombinant host cell according to claim 37, which is selected from the group consisting of bacterial cells, yeast cells, fungal cells, plant cells, insect cells, animal cells, and human cells.

58. An isolated recombinant host cell according to claim 57, which is an *E. coli* cell.

## Patentansprüche

1. Verfahren zur Erhöhung der Produktion eines ausgewählten Genprodukts in einer rekombinanten Wirtszelle umfassend:
a) das Klonieren einer Gensequenz, welche das ausgewählte Genprodukt kodiert, einer ersten Nukleinsäuresequenz, welche Thioredoxin kodiert und einer zweiren Nukleinsäuresequenz, welche Vitreoscilla-Hämoglobin kodiert, in eine Wirtszelle, um so eine rekombinante Wirtszelle zu bilden;
b) das Kultivieren der gemäß Schritt a) erzeugten rekombinanten Wirtszelle in einem geeigneten Medium, um die Expression der besagten Gensequenz zu erlauben; und
c) das Ernten des exprimierten Genprodukts.

2. Ein Verfahren gemäß Anspruch 1, wobei Schritt a) durch das Klonieren besagter Gensequenz, besagter erster Nukleinsäuresequenz und besagter zweiter Nukleinsäuresequenz zusammen in einem Vektor, der in besagter Wirtszelle exprimierbar ist und das Übertragen des so gebildeten rekombinanten Vektors in die Wirtszelle ausgeführt wird.

3. Ein Verfahren gemäß Anspruch 1, wobei Schritt a) durch unabhängiges Klonieren besagter Gensequenz, besagter erster Nukleinsäuresequenz und besagter zweiter Nukleinsäuresequenz in einen Vektor, der in besagter Wirtszelle exprimierbar ist und das Übertragen des so gebildeten rekombinanten Vektors in die Wirtszelle ausgeführt wird.

4. Ein Verfahren gemäß Anspruch 1, wobei Schritt a) ausgeführt wird durch:
i) das Konstruieren eines ersten rekombinanten Vektors, der in der Wirtszelle exprimierbar ist und das Einführen der ersten Nukleinsäuresequenz und der zweiten Nukleinsäurcsequenz;
ii) das Konstruieren eines zweiten rekombinanten Vektors, der in der Wirtszelle exprimierbar ist und das Einfügen der Gensequenz; und
iii) das Übertragen des ersten rekombinanten Vektors und des zweiten rekombinanten Vektors in die Wirtszelle.

5. Ein Verfahren gemäß Anspruch 4, wobei besagter erster rekombinanter Vektor, konstruiert gemäß dem Unterschritt i), weiterhin eine induzierbare Promotersequenz enthält, die die Expression der besagten ersten Nukleinsäuresequenz und der besagten zweiten Nukleinsäuresequenz kontrolliert.

6. Ein Verfahren gemäß Anspruch 5, wobei besagter Promotersequenz, die in besagtem ersten rekombinanten Vektor, hergestellt gemäß dem Unterschritt i) enthalten ist, abgeleitet ist aus einem der nachfolgenden: Viren, Bakterienzellen, Hefezellen, Pilzzellen, Algenzellen, Pflanzenzellen, Insektenzellen, Tierzellen und menschlichen Zellen.

7. Ein Verfahren gemäß Anspruch 6, wobei die Protnotersequenz, die in besagtem ersten rekombinanten Vektor, hergestellt gemäß Unterschritt i) enthalten ist, ausgewählt ist aus der Gruppe bestehend aus tac-Promoter, T7-Promoter, T7A1-Promoter, lac-Promoter, trp-Promoter, trc-Promoter, araBAD-Promoter und P_{R}P_{L}-Promoter.

8. Ein Verfahren gemäß Anspruch 7, wobei die Promotersequenz, die in besagtem ersten rekombinanten Vektor, konstruiert gemäß des Unterschritts i), enthalten ist, ein tac-Promoter ist.

9. Ein Verfahren gemäß Anspruch 4, wobei in besagtem ersten rekombinanten Vektor konstruiert gemäß des Unterschritts i) besagte erste Nukleinsäuresequenz und besagte zweite Nukleinsäuresequenz verbunden sind, um ein Fusionsprotein gebildet aus Thioredoxin und Hämoglobin, zu kodieren.

10. Ein Verfahren gemäß Anspruch 4, wobei besagter erster rekombinanter Vektor konstruiert gemäß Unterschritt i) weiterhin eine induzierbare erste Promotersequenz enthält, um die Expression der besagten ersten Nukleinsäuresequenz zu kontrollieren und eine induzierbare zweite Promotersequenz enthält, um die Expression der besagten zweiten Nukleinsäuresequenz zu kontrollieren, wobei besagte erste Promotersequenz und besagte zweite Promotersequenz voneinander verschieden sind.

11. Ein Verfahren gemäß Anspruch 10, wobei in besagtem ersten rekombinanten Vektor, hergestellt gemäß Unterschritt i), besagte erste Promotersequenz und besagte zweite Promotersequenz unabhängig voneinander von einem der nachfolgenden abstammen: Viren, Bakterienzellen, Hefezellen, Pilzzellen, Algenzellen, Pflanzenzellen, Insektenzellen, Tierzellen und menschlichen Zellen.

12. Ein Verfahren gemäß Anspruch 11, wobei in besagtem ersten rekombinanten Vektor, hergestellt gemäß Unterschritt i), besagte erste Promotersequenz und besagte zweite Promotersequenz unabhängig voneinander ausgewählt sind aus der Gruppe bestehend aus tac-Promoter. T7-Promoter, T7A1-Promoter, lac-Promoter, trp-Promoter, trc-Promoter, araBAD-Promoter und P_{R}P_{L}-Promoter.

13. Ein Verfahren gemäß Anspruch 11, wobei in besagtem ersten rekombinanten Vektor, hergestellt gemäß Unterschritt i), besagte erste Promotersequenz ein tac-Promoter ist und besagter zweiter Promoter ein T7A1-Promoter ist.

14. Ein Verfahren gemäß Anspruch 4, wobei besagter erster rekombinanter Vektor, hergestellt gemäß Unterschritt i), weiterhin wenigstens einen der nachfolgenden enthält: ein Marker-Gen, ein Reporter-Gen, ein antibiotisches Resistenz-Gen, eine Verstärkersequenz, eine Polyadenylierungsstelle und eine regulatorische Sequenz.

15. Ein Verfahren gemäß Anspruch 4, wobei besagte erste Nukleinsäuresequenz, verwendet im Schritt a), von dem Thioredoxin-Gen aus einer der nachfolgenden Zellen stammt: Bakterienzellen, Hefezellen, Pilzzellen, Algenzellen, Pflanzenzellen, Insektenzellen, Tierzellen und menschlichen Zellen.

16. Ein Verfahren gemäßAnspruch 15, wobei besagte erste Nukleinsäuresequenz, verwendet im Schritt a), von dem Thioredoxin-Gen (trxA) aus *E.coli* stammt.

17. Ein Verfahren gemäß Anspruch 16, wobei besagte zweite Nukleinsäuresequenz, verwendet in Schritt a), von dem Hämoglobin-Gen (VGB) von *Vitreoscilla Sp.* stammt.

18. Ein Verfahren gemäß Anspruch 1, wobei besagtes ausgewähltes Genprodukt ein homologes Polypeptid oder ein heterologes Polypeptid ist.

19. Ein Verfahren gemäß Anspruch 18, wobei besagtes ausgewähltes Genprodukt ein Enzym, ein therapeutisches Polypeptid, eine Antigendeterminante oder ein Antikörper ist.

20. Ein Verfahren gemäß Anspruch 19, wobei besagtes ausgewähltes Genprodukt ausgewählt ist aus der Gruppe bestehend aus Interferon, β-Galactosidase, Esterase und Aspertase.

21. Ein Verfahren gemäß Anspruch 1, wobei die Wirtszelle verwendet im Schritt a) ausgewählt ist aus der Gruppe bestehend aus Bakterienzellen, Helezellen, Pilzzellen, Insektenzellen, Pflanzenzellen, Tierzellen und menschlichen Zellen.

22. Ein Verfahren gemäß Anspruch 21, wobei die Wirtszelle verwendet in Schritt a) eine *E.coli*-Zelle ist.

23. Ein Nukleinsäurekonstrukt umfassend eine erste Nukleinsäuresequenz, die Thioredoxinkodiert, und eine zweite Nukleinsäurcscqucnz, die *Vitreoscilla* Hämoglobin kodiert.

24. Ein Nukleinsäurekonstrukt gemäß Anspruch 23, weiterhin umfassend eine induzierbare Promotersequenz, welche die Expression der besagten ersten Nukleinsäuresequenz und der besagten zweiten Nukleinsäuresequenz kontrolliert.

25. Ein Nukleinsäurekonstrukt gemäß Anspruch 24, wobei besagte Promotersequenz von einem der nachfolgenden stammt: Viren, Bakterienzellen, Hefezellen, Pilzzellen, Algenzellen, Pflanzenzellen, Insektenzellen, Tierzellen und menschlichen Zellen.

26. Ein Nukleinsäurekonstrukr gemäß Anspruch 25, wobei besagte Promotersequenz ausgewählt ist aus der Gruppe bestehend aus tac-Promoter, T7-Promoter, T7A1-Promoter, lac-Promoter, trp-Promoter, trc-Promoter, araBAD-Promoter und P_{R}P_{L}-Promoter.

27. Ein Nukleinsäurekowtrukt gemäß Anspruch 25, wobei besagte Promotersequenz ein tac-Promoter ist.

28. Ein Nukleinsäurekonstrukt gemäß Anspruch 24, wobei besagte erste Nukleinsäuresequenz und besagte zweite Nukleinsäuresequenz verbunden sind, um ein Fusionsprotein gebildet aus Thioredoxin und Hämoglobin zu kodieren.

29. Ein Nukleinsäurckonstrukt gemäß Anspruch 23, weiterhin umfassend eine induzierbare erste Promotersequenz, um die Expression der besagten ersten Nukleinsäuresequenz zu kontrollieren und eine induzierbare zweite Promotersequenz, um die Expression der besagten zweiten Nukleinsäuresequenz zu kontrollieren, wobei besagte erste Promotersequenz und besagte zweite Promotersequenz voneinander verschieden sind.

30. Ein Nukleinsäurekonstrukt gemäß Anspruch 29, wobei besagte erste Promotersequenz und besagte zweite Promotersequenz unabhängig voneinander von einem der nachfolgenden stammen: Viren, Bakterienzellen, Hefezellen, Pilzzellen, Algenzellen, Pflanzenzellen, Insektenzellen, Tierzellen und menschlichen Zellen.

31. Ein Nukleinsäurekonstrukt gemäß Anspruch 30, wobei besagte erste Promotersequenz und besagte zweite Promotersequenz unabhängig voneinander ausgewählt sind aus der Gruppe bestehend aus tac-Promoter, T7-Promoter, T7A1-Promoter, lac-Promoter, trp-Promoter, trc-Promoter, araRAD-Promoter und P_{R}P_{L}-Promoter.

32. Ein Nukleinsäurekonstrukt gemäß Anspruch 30, wobei besagte erste Promotersequenz ein tac-Promoter ist und besagte zweite Promotersequenz ein T7A1-Promoter ist.

33. Ein Nukleinsäurekonstrukt gemäß Anspruch 23, wobei besagte erste Nukleinsäuresequenz von dem Thiorcdoxin-Gen einer der nachfolgenden Zellen stammt: Bakterienzellen, Hefezellen, Pilzzellen, Algenzellen, Pflanzenzellen, Insektenzellen, Tierzellen und menschlichen Zellen.

34. Ein Nukleinsäurekonstrukt gemäß Anspruch 33, wobei besagte erste Nukleinsäuresequenz von dem Thioredoxin-Gen (trxA) aus *E.coli* stammt.

35. Ein Vektor umfassend ein Nukleinsäurekonstrukt gemäß einem der Ansprüche 24 bis 34.

36. Ein Vektor gemäß Anspruch 35, weiter umfassend wenigstens eines der Folgenden: ein Marker-Gen, ein Reporter-Gen, ein antibiotisches Resistenz-Gen, eine Verstärkersequenz, ein Gen, welches ein ausgewähltes Genprodukt kodiert, eine Polyadenylation-Stelle und eine regulatorische Sequenz.

37. Eine isolierte rekombinante Wirtszelle, die in der Lage ist, ein ausgewähltes Genprodukt zu exprimieren, umfassend eine Gensequenz, die für besagtes ausgewähltes Genprodukt kodiert, eine erste Nukleinsäuresequenz, die Thioredoxin kodiert, und eine zweite Nukleinsäuresequenz, die *Vitreoscilla*-Hämoglobin kodiert.

38. Eine isolierte rekombinante Wirtszelle gemäß Anspruch 37, wobei besagte Gensequenz, besagte erste Nukleinsäuresequenz und besagte zweite Nukleinsäuresequenz gemeinsam von einem Vektor, der in besagter Wirtszelle exprimierbar ist, getragen werden.

39. Eine isolierte rekombinante Wirtszelle gemäß Anspruch 37, wobei besagte Gensequenz, besagte erste Nukleinsäuresequenz und besagte zweite Nukleinsäuresequenz unabhängig voneinander von einem Vektor, der in besagter Wirtszelle exprimierbar ist, getragen werden.

40. Eine isolierte rekombinante Wirtszelle gemäß Anspruch 37, wobei besagte erste Nukleinsäuresequenz und besagte zweite Nukleinsäuresequenz von einem ersten Vektor, der in besagter Wirtszelle exprimierbar ist, getragen werden und besagte Gensequenz von einem zweiten Vektor, der in besagter Wirtszelle exprimierbar ist, getragen wird.

41. Eine isolierte rekombinante Wirtszelle gemäß Anspruch 40, wobei besagter erster Vektor eine indizierbare Promotersequenz umfasst, um die Expression der besagten ersten Nukleinsäuresequenz und der besagten zweiten Nukleinsäuresequenz zu kontrollieren.

42. Eine isolierte rekombinante Wirtszelle gemäß Anspruch 41, wobei besagte Promotersequenz von einem der nachfolgenden stammt: Viren, Bakterienzellen, Hefezellen, Pilzzellen, Algenzellen, Pflanzenzellen, Insektenzellen, Tierzellen und menschlichen Zellen.

43. Eine isolierte rekombinante Wirtszelle gemäß Anspruch 42, wobei besagte Promotersequcnz ausgewählt ist aus der Gruppe bestehend aus tac-Promoter, T7-Promoter, T7A1-Promoter, lac-Promoter, trp-Promoter, trc-Promoter, araBAD-Promoter und P_{R}P_{L}-Promoter.

44. Eine isolierte rekombinante Wirtszelle gemäß Anspruch 43, wobei besagte Promotersequenz ein tac-Promoter ist.

45. Eine isolierte rekombinante Wirtszelle gemäß Anspruch 40, wobei besagte erste Nukleinsäuresequenz und besagte zweite Nukleinsäuresequenz verbunden sind, um ein Fusionsprotein gebildet aus Thioredoxin und Hämoglobin zu kodieren.

46. Eine isolierte rekombinante Wirtszelle gemäß Anspruch 40, wobei besagter erster Vektor weiterhin eine induzierbare erste Promotersequenz umfasst, um die Expression der besagten ersten Nukleinsäuresequenz zu kontrollieren und eine induzierbare zweite Promotersequenz umfasst, um die Expression der besagten zweiten Nukleinsäuresequenz zu kontrollieren, wobei besagte erste Promotersequenz und besagter zweite Promoter voneinander verschieden sind.

47. Eine isolierte rekombinante Wirtszelle gemäß Anspruch 46, wobei besagte erste Promotersequenz und besagte zweite Promotersequenz unabhängig voneinander von einem der nachfolgenden stammen: Viren, Bakterienzellen, Hefezellen, Pilzzellen, Algenzellen, Pflanzenzellen, Insektenzellen, Tierzellen und menschlichen Zellen.

48. Eine isolierte rekombinante Wirtszelle gemäß Anspruch 47, wobei besagte erste Promotersequenz und besagte zweite Promotersequenz unabhängig voneinander ausgewählt sind aus der Gruppe bestehend aus tac-Promoter, T7-Promoter, T7A1-Promoter, lac-Promoter, trp-Promoter, trc-Promoter, araBAD-Promoter und P_{R}P_{L}-Promoter.

49. Eine isolierte rekombinante Wirtszelle gemäß Anspruch 48, wobei besagte erste Promotersequenz ein tac-Promoter ist und besagter zweiter Promoter ein T7A1-Promoter ist.

50. Eine isolierte rekombinante Wirtszelle gemäß Anspruch 37, wobei besagte erste Nukleinsäuresequenz von dem Thioredoxin-Gen aus einer der folgenden Zellen stammt: Bakterienzellen, Hefezellen, Pilzzellen, Algenzellen, Pflanzenzellen, Insektenzellen, Tierzellen und menschlichen Zellen.

51. Eine isolierte rekombinante Wirtszelle gemäß Anspruch 50, wobei besagte erste Nukleinsäuresequenz von dem Thioredoxin-Gen (trxA) aus *E.coli* stammt.

52. Eine isolierte rekombinante Wirtszelle gemäß Anspruch 40, wobei besagter erster Vektor weiterhin wenigstens eines aus ein Marker-Gen, ein Reporrer-Gen, ein antibiotisches Resistenz-Gen, eine Verstärkersequenz, eine Polyadenylierungsscelle und eine regulatorische Sequenz enthält.

53. Eine isolierte rekombinante Wirtszelle gemäß Anspruch 37, wobei besagte erste Nukleinsäuresequenz und besagte zweite Nukleinsäuresequenz in die genomische DNA der besagten Wirtszelle eingebaut sind.

54. Eine isolierte rekombinante Wirtszelle gemäß Anspruch 37, wobei besagtes ausgewähltes Genprodukt ein homologes Polypeptid oder ein heterologes Polypeptid ist.

55. Eine isolierte rekombinante Wirtszelle gemäß Anspruch 54, wobei besagtes ausgewähltes Genprodukt ein Enzym, ein therapeutisches Polypeptid, eine Antigendererminante oder ein Antikörper ist.

56. Eine isolierte rekombinante Wirtszelle gemäß Anspruch 55, wobei besagtes ausgewähltes Genprodukt ausgewählt ist aus der Gruppe bestehend aus Interferon, β-Galactosidase, Esterase und Aspertase.

57. Eine isolierte rekombinante Wirtszelle gemäß Anspruch 37, die ausgewählt aus der Gruppe bestehend aus Bakterienzellen, Hefezellen, Pilzzellen, Pflanzenzellen, Insektenzellen, Tierzellen und menschlichen Zellen.

58. Eine isolierte rekombinante Wirtszelle gemäß Anspruch 57, die eine *E.coli*-Zelle ist.

## Revendications

1. Un procédé pour améliorer la production d'un produit génétique sélectionné dans une cellule hôte recombinante comprenant :
(a) clonage dans une cellule hôte d'une séquence génétique encodant le produit génétique sélectionné, d'une première séquence d'acide nucléique encodant la thioredoxine et d'une seconde séquence d'acide nucléique encodant l'hémoglobine Vitreoscilla, de sorte à former une cellule hôte recombinante ;
(b) culture d'une cellule hôte recombinante formée par l'étape (a) dans un médium adapté de sorte à permettre l'expression de ladite séquence génétique et
(c) récolte produit génétique exprimé.

2. Un procédé selon la revendication 1 où l'étape (a) est conduite par clonage de ladite séquence génétique, ladite première séquence d'acide nucléique et ladite seconde séquence d'acide nucléique ensemble dans un vecteur exprimable dans ladite cellule hôte et transfert du vecteur recombinant ainsi formé dans la cellule hôte.

3. Un procédé selon la revendication 1 où l'étape (a) est conduite en clonant indépendamment ladite séquence génétique, ladite première séquence d'acide nucléique et ladite seconde séquence d'acide nucléique dans un vecteur exprimable dans ladite cellule hôte et transfert du vecteur recombinant ainsi formé dans la cellule hôte.

4. Un procédé selon la revendication 1 où l'étape (a) est conduite par
(i) construction d'un premier vecteur recombinant exprimable dans la cellule hôte et inclusion d'une première séquence d'acide nucléique et d'une seconde séquence d'acide nucléique ;
(ii) construction d'un second vecteur recombinant exprimable dans la cellule hôte et inclusion de la séquence génétique ; et
(iii) transfert du premier vecteur recombinant et du second vecteur recombinant dans la cellule hôte.

5. Un procédé selon la revendication 4 où ledit premier vecteur recombinant construit à partir de la sous étape (i) inclut de plus une séquence promotrice inductible pour contrôler l'expression de ladite première séquence d'acide nucléique et de ladite seconde séquence d'acide nucléique.

6. Un procédé selon la revendication 5 où ladite séquence promotrice inclue dans ledit premier vecteur recombinant construit à partir de la sous étape (i) est dérivée de l'un des suivants : virus, cellules bactériennes, cellules de levure, cellules fongiques, cellules algales, cellule végétales, cellules d'insecte, cellules animales, et cellules humaines.

7. Un procédé selon la revendication 6 où ladite séquence promotrice inclue dans ledit premier vecteur recombinant construit à partir de la sous étape (i) est sélectionné parmi le groupe constitué de promoteur tac, promoteur T7, promoteur T7A1, promoteur lac, promoteur trp, promoteur trc, promoteur araBAD, et promoteur P_{R}P_{L}.

8. Un procédé selon la revendication 7 où ladite séquence promotrice inclue dans ledit premier vecteur recombinant construit à partir de la sous-étape (i) est un promoteur tac.

9. Un procédé selon la revendication 4 où, dans ledit premier vecteur recombinant construit à partir de la sous étape (i), ladite première séquence d'acide nucléique et ladite seconde séquence d'acide nucléique sont connectées pour encoder une protéine de fusion formée de thioredoxine et hémoglobine.

10. Un procédé selon la revendication 4 où ledit premier vecteur recombinant construit à partir de la sous étape (i) inclut de plus une première séquence promotrice inductible pour contrôler l'expression de ladite première séquence d'acide nucléique et une seconde séquence promotrice inductible pour contrôler l'expression de ladite seconde séquence d'acide nucléique, ladite première séquence promotrice et ladite seconde séquence promotrice étant différente l'une de l'autre.

11. Un procédé selon la revendication 10, où dans ledit premier vecteur recombinant construit à partir de la sous étape (i) ladite première séquence promotrice et ladite seconde séquence promotrice sont indépendamment dérivées de l'un des suivants : virus, cellules bactériennes, cellules de levure, cellules fongiques, cellules algales, cellule végétales, cellules d'insecte, cellules animales, et cellules humaines.

12. Un procédé selon la revendication 11, où dans ledit premier vecteur recombinant construit à partir de la sous étape (i) ladite première séquence promotrice et ladite seconde séquence promotrice sont indépendamment sélectionnées parmi le groupe constitué de promoteur tac, promoteur T7, promoteur T7A1, promoteur lac, promoteur trp, promoteur trc, promoteur araBAD, et promoteur P_{R}P_{L}.

13. Un procédé selon la revendication 11 où dans ledit premier vecteur recombinant construit à partir de la sous étape (i) ladite première séquence promotrice est un promoteur tac et ladite seconde séquence promotrice est un promoteur T7A1.

14. Un procédé selon la revendication 4, où ledit premier vecteur recombinant construit à partir de la sous étape (i) comprend de plus au moins l'un des suivants : gène marqueur, gène rapporteur, gène d'antibiorésistance, une séquence activatrice, un site de polyadénylation, et une séquence régulatrice.

15. Un procédé selon la revendication 4 où ladite première séquence d'acide nucléique utilisée en étape (a) est dérivée du gêne de thioredoxine de toute cellule parmi les suivantes : cellules bactériennes, cellules de levure, cellules fongiques, cellules algales, cellule végétales, cellules d'insecte, cellules animales, et cellules humaines.

16. Un procédé selon la revendication 15 où ladite première séquence d'acide nucléique utilisée en étape (a) est dérivée du gêne de thioredoxine (trxA) de E. coli.

17. Un procédé selon la revendication 16 où ladite seconde séquence d'acide nucléique utilisée en étape (a) est dérivée du gêne d'hémoglobine (vgb) de Vitreoscilla sp.

18. Un procédé selon la revendication 1 où ledit produit génétique sélectionné est un polypeptide homologue ou polypeptide hétérologue.

19. Un procédé selon la revendication 18 où ledit produit génétique sélectionné est une enzyme, un polypeptide thérapeutique, un déterminant antigénique ou un anticorps.

20. Un procédé selon la revendication 19 où ledit produit génétique sélectionné est sélectionné parmi le groupe constitué d'interféron, β galactosidase, estérase et aspartase.

21. Un procédé selon la revendication 1 où la cellule hôte utilisée en étape (a) est sélectionnée parmi le groupe constitué de cellules bactériologiques cellules bactériennes, cellules de levure, cellules fongiques, cellules algales, cellule végétales, cellules d'insecte, cellules animales, et cellules humaines.

22. Un procédé selon la revendication 21 où la cellule hôte utilisée en étape (a) est une cellule E. coli.

23. Une construction d'acide nucléique comprenant une première séquence d'acide nucléique encodant la thioredoxine et une seconde séquence d'acide nucléique encodant l'hémoglobine Vitreoscilla.

24. Une construction d'acide nucléique selon la revendication 23 comprenant de plus une séquence promotrice inductible pour contrôler l'expression de ladite première séquence d'acide nucléique et de ladite seconde séquence d'acide nucléique.

25. Une construction d'acide nucléique selon la revendication 24 où ladite séquence promotrice est dérivée de virus, cellules bactériennes, cellules de levure, cellules fongiques, cellules algales, cellule végétales, cellules d'insecte, cellules animales, et cellules humaines.

26. Une construction d'acide nucléique selon la revendication 25 où ladite séquence promotrice est sélectionnée parmi le groupe constitué de promoteur tac, promoteur T7, promoteur T7A1, promoteur lac, promoteur trp, promoteur trc, promoteur araBAD, et promoteur PᵣP_{L}.

27. Une construction d'acide nucléique selon la revendication 25 où ladite séquence promotrice est un promoteur tac.

28. Une construction d'acide nucléique selon la revendication 24 où ladite première séquence d'acide nucléique et ladite seconde séquence d'acide nucléique sont connectées pour encoder une protéine de fusion formée de thioredoxide et hémoglobine.

29. Une construction d'acide nucléique selon la revendication 23 comprenant de plus une première séquence promotrice inductible pour contrôler l'expression de ladite première séquence d'acide nucléique et une seconde séquence promotrice inductible pour contrôler l'expression de ladite seconde séquence d'acide nucléique, ladite première séquence promotrice et ladite seconde séquence promotrice étant différente l'une de l'autre.

30. Une construction d'acide nucléique selon la revendication 29 où ladite première séquence promotrice et ladite seconde séquence promotrice sont indépendamment dérivées de l'un des suivants : virus, cellules bactériennes, cellules de levure, cellules fongiques, cellules algales, cellule végétales, cellules d'insecte, cellules animales, et cellules humaines.

31. Une construction d'acide nucléique selon la revendication 30 où ladite première séquence promotrice et ladite seconde séquence promotrice sont indépendamment sélectionnées parmi le groupe constitué de promoteur tac, promoteur T7, promoteur T7A1, promoteur lac, promoteur trp, promoteur trc, promoteur araBAD, et promoteur PᵣP_{L}.

32. Une construction d'acide nucléique selon la revendication 30 où ladite première séquence promotrice est un promoteur tac et ladite seconde séquence promotrice est un promoteur T7A1.

33. Une construction d'acide nucléique selon la revendication 23 où ladite première séquence d'acide nucléique utilisée en étape (a) est dérivée du gène de thioredoxine de toute cellule parmi les suivantes : cellules bactériennes, cellules de levure, cellules fongiques, cellules algales, cellule végétales, cellules d'insecte, cellules animales, et cellules humaines.

34. Une construction d'acide nucléique selon la revendication 33 où ladite première séquence d'acide nucléique utilisée en étape (a) est dérivée du gène de thioredoxine (trxA) de E. coli.

35. Un vecteur comprenant la construction d'acide nucléique selon l'une des revendications 24-34.

36. Un vecteur selon la revendication 35 comprenant de plus au moins l'un des suivants : gêne marqueur, gène rapporteur, gène d'antibiorésistance, une séquence activatrice, un gène encodant un produit génétique sélectionné, un site de polyadénylation, et une séquence régulatrice.

37. Une cellule hôte recombinante isolée capable d'exprimer un produit génétique sélectionné, comprenant une séquence génétique encodant ledit produit génétique, une première séquence d'acide nucléique encodant la thioredoxine et une seconde séquence d'acide nucléique encodant l'hémoglobine Vitreoscilla.

38. Une cellule hôte recombinante isolée selon la revendication 37 où ladite séquence génétique, ladite première séquence d'acide nucléique et ladite seconde séquence d'acide nucléique ensemble sont portées dans un vecteur exprimable dans ladite cellule hôte.

39. Une cellule hôte recombinante isolée selon la revendication 37 où ladite séquence génétique, ladite première séquence d'acide nucléique et ladite seconde séquence d'acide nucléique sont portées indépendamment dans un vecteur exprimable dans ladite cellule hôte.

40. Une cellule hôte recombinante isolée selon la revendication 37 où ladite première séquence d'acide nucléique et ladite seconde séquence d'acide nucléique sont portées dans un premier vecteur exprimable dans ladite cellule hôte et où ladite séquence génétique est portée dans un second vecteur exprimable dans ladite cellule hôte.

41. Une cellule hôte recombinante isolée selon la revendication 40 où ledit premier vecteur comprend de plus une séquence promotrice inductible pour contrôler l'expression de ladite première séquence d'acide nucléique et de ladite seconde séquence d'acide nucléique.

42. Une cellule hôte recombinante isolée selon la revendication 41 où ladite séquence promotrice est dérivée de l'un des suivants : virus, cellules bactériennes, cellules de levure, cellules fongiques, cellules algales, cellule végétales, cellules d'insecte, cellules animales, et cellules humaines.

43. Une cellule hôte recombinante isolée selon la revendication 42 où ladite séquence promotrice est sélectionnée parmi le groupe constitué de promoteur tac, promoteur T7, promoteur T7A1, promoteur lac, promoteur trp, promoteur trc, promoteur araBAD, et promoteur PᵣP_{L}.

44. Une cellule hôte recombinante isolée selon la revendication 43 ladite séquence promotrice est un promoteur tac.

45. Une cellule hôte recombinante isolée selon la revendication 40 où ladite première séquence d'acide nucléique et ladite seconde séquence d'acide nucléique sont connectées pour encoder une protéine de fusion formée de thioredoxide et hémoglobine.

46. Une cellule hôte recombinante isolée selon la revendication 40 où ledit premier vecteur comprend une première séquence promotrice inductible pour contrôler l'expression de ladite première séquence d'acide nucléique et une seconde séquence promotrice inductible pour contrôler l'expression de ladite seconde séquence d'acide nucléique, ladite première séquence promotrice et ladite seconde séquence promotrice étant différente l'une de l'autre.

47. Une cellule hôte recombinante isolée selon la revendication 46 où ladite première séquence promotrice et ladite seconde séquence promotrice sont indépendamment dérivées de l'un des suivants : virus, cellules bactériennes, cellules de levure, cellules fongique, cellules algales, cellule végétales, cellules d'insecte, cellules animales, et cellules humaines.

48. Une cellule hôte recombinante isolée selon la revendication 47 où ladite première séquence promotrice et ladite seconde séquence promotrice sont indépendamment sélectionnées parmi le groupe constitué de promoteur tac, promoteur T7, promoteur T7A1, promoteur lac, promoteur trp, promoteur trc, promoteur araBAD, et promoteur PᵣP_{L}.

49. Une cellule hôte recombinante isolée selon la revendication 48 où ladite première séquence promotrice est un promoteur tac et ladite seconde séquence promotrice est un promoteur T7A1.

50. Une cellule hôte recombinante isolée selon la revendication 37 où ladite première séquence d'acide nucléique est dérivée du gène de thioredoxine de toute cellule parmi les suivantes : cellules bactériennes, cellules de levure, cellules fongiques, cellules algales, cellule végétales, cellules d'insecte, cellules animales, et cellules humaines.

51. Une cellule hôte recombinante isolée selon la revendication 50 où ladite première séquence d'acide nucléique est dérivée du gène de thioredoxine (trxA) de E. coli.

52. Une cellule hôte recombinante isolée selon la revendication 40 où ledit premier vecteur comprend de plus au moins un parmi gène marqueur, gène rapporteur, gène d'antibiorésistance, une séquence activatrice, un gène encodant un produit génétique sélectionné, un site de polyadénylation, et une séquence régulatrice.

53. Une cellule hôte recombinante isolée selon la revendication 37 où ladite première séquence d'acide nucléique et ladite seconde séquence d'acide nucléique sont incorporées dans l'ADN génomique de ladite cellule hôte.

54. Une cellule hôte recombinante isolée selon la revendication 37 où ladite produit génétique sélectionné est un polypeptide homologue ou polypeptide hétérologue.

55. Une cellule hôte recombinante isolée selon la revendication 54 où ledit produit génétique sélectionné est une enzyme, un polypeptide thérapeutique, un déterminant antigénique ou un anticorps.

56. Une cellule hôte recombinante isolée selon la revendication 55 où ledit produit génétique sélectionné est sélectionné parmi le groupie constitué d'interféron, β galactosidase, estérase et aspartase.

57. Une cellule hôte recombinante isolée selon la revendication 37 qui est sélectionnée parmi le groupe constituée de cellules bactériennes, cellules de levure, cellules fongiques, cellules algales, cellule végétales, cellules d'insecte, cellules animales, et cellules humaines.

58. Une cellule hôte recombinante isolée selon la revendication 57 qui est une cellule E.coli.
